(19) Europäisches Patentamt / European Patent Office / Office européen des brevets

(11) **EP 4 548 049 B1**

## (12) FASCICULE DE BREVET EUROPEEN

(45) Date de publication et mention
de la délivrance du brevet:
**27.05.2026   Bulletin 2026/22**

(21) Numéro de dépôt: **23734678.8**

(22) Date de dépôt: **26.06.2023**

(51) Classification Internationale des Brevets (IPC):
*G01F 1/64* (2006.01)        *A61B 5/145* (2006.01)
*G01F 1/704* (2006.01)       *G01F 1/7088* (2022.01)
*G01F 1/712* (2006.01)       *A61B 5/00* (2006.01)
*A61B 5/1477* (2006.01)      *A61B 5/11* (2006.01)

(52) Classification Coopérative des Brevets (CPC):
**G01F 1/64; A61B 5/1118; A61B 5/4266;**
**A61B 5/6823; A61B 5/7246; G01F 1/704;**
**G01F 1/7046; G01F 1/7088; G01F 1/712;**
A61B 5/14517; A61B 5/1477; A61B 2562/0219

(86) Numéro de dépôt international:
**PCT/EP2023/067355**

(87) Numéro de publication internationale:
**WO 2024/002988 (04.01.2024 Gazette 2024/01)**

(54) **DISPOSITIF ÉLECTROCHIMIQUE MICROFLUIDIQUE DE MESURE D'UN DÉBIT VOLUMIQUE**

MIKROFLUIDISCHE ELEKTROCHEMISCHE VORRICHTUNG ZUR MESSUNG EINES
VOLUMENSTROMS

MICROFLUIDIC ELECTROCHEMICAL DEVICE FOR MEASURING A VOLUME FLOW RATE

(84) Etats contractants désignés:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Etats de validation désignés:
**MA TN**

(30) Priorité:  **29.06.2022   FR 2206577**

(43) Date de publication de la demande:
**07.05.2025   Bulletin 2025/19**

(73) Titulaire: **NOPTRACK**
**81100 Castres (FR)**

(72) Inventeurs:
• **BEDIOUI, Fethi**
**75010 PARIS (FR)**

• **DELAHAYE, Thomas**
**14120 MONDEVILLE (FR)**
• **GRIVEAU, Sophie**
**91300 MASSY (FR)**
• **SELLA, Catherine**
**92360 Meudon la forêt (FR)**
• **THOUIN, Laurent**
**92160 ANTONY (FR)**
• **AMATORE, Christian**
**75013 PARIS (FR)**
• **FAVRE, Gilles**
**31270 CUGNAUX (FR)**

(74) Mandataire: **Loyer & Abello**
**9, rue Anatole de la Forge**
**75017 Paris (FR)**

(56) Documents cités:
**WO-A1-2021/105122      US-A1- 2008 257 063**

## Description

### Domaine technique

L'invention se rapporte au domaine des dispositifs électrochimiques microfluidiques et aux procédés permettant de mesurer un débit volumique d'un fluide électroactif ou contenant une ou plusieurs espèces électroactives dans un canal microfluidique. Plus généralement, l'invention se rapporte également à un appareil pour déterminer un paramètre quantitatif de sudation d'un sujet, humain ou animal.

### Arrière-plan technologique

La sueur est sécrétée par les glandes sudoripares de la peau, évacuée par les pores de la peau et s'évapore au niveau de l'épiderme. La sudation joue un rôle important pour l'organisme du sujet puisqu'elle permet la thermorégulation corporelle par transpiration. Une sudation excessive peut entraîner la déshydratation du sujet, altérer ses performances physiques, et conduire à des conséquences délétères pour sa santé. De même, une consommation excessive d'eau peut entraîner l'hyponatrémie, la fatigue, la confusion, coma, voire la mort du sujet.

On connaît dans l'état de l'art différents dispositifs de mesure *in situ* de micro-débits, notamment des capteurs de flux thermiques ou encore des micro-débitmètres à effet Coriolis. En pratique, ces dispositifs sont coûteux, présentent des problèmes de fiabilité non résolus, et sont généralement incorporés dans des boîtiers encombrants. Plus particulièrement, des dispositifs microfluidiques ont été développés pour mesurer les micro-débits de sueur transpirée par un sujet, humain ou animal, circulant dans des canaux microfluidiques. La mesure d'un micro-débit de sueur permet d'évaluer un paramètre quantitatif de sudation du sujet pour, par exemple, suivre le niveau d'hydratation du sujet afin de prévenir les déséquilibres hydriques du corps, en particulier chez les sportifs et les personnes âgées, ou encore diagnostiquer l'hypohidrose, un trouble de la sudation qui se caractérise par une insuffisance de sueur et qui peut être causé par des pathologies susceptibles d'endommager le fonctionnement des glandes sudoripares (le diabète, l'alcoolisme, la maladie de Parkinson, le syndrome de Ross, le syndrome de Sjögren, les cancers du poumon à petites cellules, etc.), par des causes cutanées (les brûlures, les inflammations, les infections, les pathologies cutanées, etc.), par des causes médicamenteuses (e.g., les traitements par anticholinergiques) et des causes génétiques (e.g., la dysplasie ectodermique hypohidrotique).

Les dispositifs microfluidiques connus présentent l'avantage de pouvoir collecter facilement et sans évaporation la sueur dans les canaux microfluidiques avec une très grande résolution temporelle. Les techniques par colorimétrie sont généralement privilégiées en raison de la facilité de fabrication des dispositifs associés. Néanmoins, leur principal inconvénient concerne le caractère irréversible du procédé. Après le remplissage du canal microfluidique par la sueur, ces dispositifs sont définitivement modifiés par les colorants et ne peuvent plus être utilisés. Cette situation est comparable aux techniques de détection basées sur des signaux de transduction électrique de type résistance, conductance, capacité ou impédance. Pour estimer les micro-débits de sueur, leur mise en œuvre consiste à suivre le taux de remplissage des canaux au moyen d'électrodes disposées le long des canaux microfluidiques. Une fois remplis, les canaux microfluidiques ne peuvent donc plus être utilisés.

Le document FR3103901A1 ou WO 2021/105122 A1 décrit notamment un procédé de mesure d'une vitesse d'écoulement de sueur basé sur un retard entre les variations temporelles de signaux ampérométriques qui représentent intrinsèquement la concentration en peroxyde d'hydrogène $H_2O_2$, monoxyde d'azote NO ou en ion nitrite $NO_2^-$, présents dans le flux de sueur.

### Résumé

Une idée à la base de l'invention est de fournir un dispositif électrochimique microfluidique pour mesurer le débit volumique d'un fluide dans un canal microfluidique, sans nécessairement effectuer la détermination de concentration d'espèces chimiques électroactives contenues dans le flux de fluide.

Une autre idée à la base de l'invention est de fournir un appareil souple et à coller sur la peau pour déterminer un paramètre quantitatif de sudation d'un sujet à partir de la mesure *in situ* et en continu du débit volumique de sueur s'écoulant dans un canal microfluidique.

Un but de l'invention est de fournir un tel appareil présentant en outre les avantages d'être peu encombrant, de conception simple et d'un coût limité.

Selon un mode de réalisation, l'invention fournit un dispositif électrochimique microfluidique pour mesurer une vitesse d'écoulement et/ou un débit volumique d'un fluide, le fluide comportant un solvant, le dispositif électrochimique microfluidique comportant :

- au moins un canal microfluidique configuré pour permettre au fluide de s'écouler selon un sens d'écoulement ;

- au moins une cellule électrochimique disposée dans l'au moins un canal microfluidique, la cellule électrochimique comportant une première électrode de travail et au moins une deuxième électrode de travail espacée de la première électrode de travail d'une distance inter-électrodes dans le sens d'écoulement, au moins une contre-électrode et au moins une électrode de référence ; et

- un système de mesure électrochimique par ampérométrie configuré pour polariser la première électrode de travail à un premier potentiel d'électrode et la deuxième électrode de travail à un deuxième potentiel d'électrode, de sorte que chacune desdites première et deuxième électrode de travail produit un signal ampérométrique par réaction d'oxydation ou par réaction de réduction du solvant ou avec au moins une espèce chimique formant un couple d'oxydoréduction avec le solvant ;

le système de mesure électrochimique par ampérométrie étant configuré pour déterminer la vitesse d'écoulement et/ou le débit volumique du fluide dans le canal microfluidique à partir de la distance inter-électrodes entre les première et deuxième électrodes de travail, et d'un retard temporel entre une variation du signal ampérométrique produit par la première électrode de travail et une variation du signal ampérométrique produit par la deuxième électrode de travail.

Un tel dispositif électrochimique microfluidique peut être incorporé dans de nombreux microsystèmes pour mesurer *in situ* le débit volumique et/ou la vitesse d'écoulement d'un fluide dans un canal microfluidique. Ces microsystèmes peuvent être par exemple des plateformes microfluidiques de type laboratoires-sur-puce (*lab - on-a-chip*) ou des microsystèmes d'analyse totale (*micro-Total Analysis System* ou $\mu$TAS).

Le dispositif électrochimique microfluidique est simple à fabriquer et facilement industrialisable, car sans pièce mobile ni aucune hypothèse sur le régime hydrodynamique de l'écoulement du fluide dans le canal microfluidique. La détermination de la vitesse d'écoulement et/ou du débit volumique n'est aucunement liée à la détermination de concentration d'espèces chimiques générées ou contenues dans le fluide, mais uniquement à un délai de réponse entre les variations des signaux ampérométriques d'une paire d'électrodes de travail.

Selon des modes de réalisation, un tel dispositif électrochimique microfluidique peut comporter une ou plusieurs des caractéristiques suivantes.

Selon un mode de réalisation, le solvant est de l'eau $H_2O_{(1)}$.

L'eau peut intervenir comme espèce chimique réductrice dans le couple d'oxydoréduction $O_2/H_2O$ et comme espèce chimique oxydante dans le couple d'oxydoréduction $H_3O^+/H_2$.

Selon un mode de réalisation, le fluide est une sueur d'un sujet humain ou animal.

Selon un mode de réalisation, le premier potentiel d'électrode permet l'oxydation de l'eau $H_2O_{(1)}$ en dioxygène $O_{2(aq)}$ et le deuxième potentiel d'électrode permet la réduction du dioxygène $O_{2(aq)}$ dissous dans l'eau $H_2O_{(1)}$ produit en eau $H_2O_{(1)}$..

Le choix des réactions d'oxydation de l'eau $H_2O_{(1)}$ à la première électrode de travail et de réduction du dioxygène $O_{2(aq)}$ à la deuxième électrode de travail, grâce aux potentiels adéquats appliqués aux première et deuxième électrodes de travail, permet de contrôler l'amplitude des signaux ampérométriques détectés à chacune desdites électrodes de travail sans nécessairement mesurer ces amplitudes mais de sorte à maintenir un rapport signal sur bruit suffisant pour permettre une détection aisée des variations des signaux ampérométriques.

Selon un mode de réalisation, le premier potentiel d'électrode permet la réduction de l'eau $H_2O_{(1)}$ en dihydrogène $H_{2(aq)}$ et le deuxième potentiel d'électrode permet la réduction de l'eau $H_2O_{(1)}$ en dihydrogène $H_{2(aq)}$.

Selon un mode de réalisation, le premier potentiel d'électrode permet la réduction du dioxygène $O_{2(aq)}$ dissous dans l'eau $H_2O_{(1)}$ en eau $H_2O_{(1)}$ et le deuxième potentiel d'électrode permet la réduction du dioxygène $O_{2(aq)}$ dissous dans l'eau $H_2O_{(1)}$ en eau $H_2 O_{(1)}$.

Selon un mode de réalisation, le système de mesure électrochimique par ampérométrie est également configuré pour :

- au cours d'une première étape, polariser la première électrode de travail au premier potentiel d'électrode et la deuxième électrode de travail au deuxième potentiel d'électrode ;

- au cours d'une deuxième étape, déconnecter la première électrode de travail ou fixer le premier potentiel d'électrode à un potentiel proche ou égal d'un potentiel d'équilibre à courant nul.

Selon un mode de réalisation, le dispositif électrochimique microfluidique comporte en outre un support isolant, ledit au moins un canal microfluidique étant formé dans le support isolant, les première et deuxième électrodes de travail étant formées par des dépôts métalliques de platine ou de noir de platine sur ledit support isolant.

Selon un mode de réalisation, la contre-électrode est positionnée en aval des électrodes de travail selon le sens d'écoulement, et dans lequel l'électrode de référence est positionnée en amont desdites électrodes de travail selon ledit sens d'écoulement.

Ainsi, l'électrode de référence est située en amont de la paire d'électrodes de travail afin de préserver la stabilité du potentiel d'électrode de référence au cours du temps ; et la contre-électrode est située en aval de la paire d'électrodes de travail et, partant, de l'électrode de référence, de sorte que les espèces chimiques générées à sa surface ne perturbent ni les électrodes de travail ni l'électrode de référence.

Avantageusement, la surface de la contre-électrode est deux à trois fois plus grande que celles des autres électrodes.

Selon des modes de réalisation, le dispositif électrochimique microfluidique peut comporter un ou plusieurs canaux microfluidiques. Le cas échéant, une cellule électrochimique peut être disposée dans un ou chaque canal microfluidique ou dans certains des ou tous les canaux microfluidiques. Les cellules électrochimiques disposées dans des canaux différents peuvent être différentes ou identiques. Les réactions d'oxydoréduction mises en œuvre dans les cellules électrochimiques disposées dans des canaux différents peuvent être différentes ou identiques.

Selon un mode de réalisation, le dispositif électrochimique microfluidique comporte un premier et un deuxième canal microfluidique, la première, respectivement, la deuxième, cellule électrochimique étant disposée dans le premier, respectivement, le deuxième, canal microfluidique, la distance inter-électrodes de la première cellule électrochimique différant de la distance inter-électrodes de la deuxième cellule électrochimique.

Selon un mode de réalisation, ladite au moins une cellule électrochimique comporte deux deuxièmes électrodes de travail respectivement séparées de la première électrode de travail par une première distance inter-électrode et par une deuxième distance inter-électrode, la première distance inter-électrode étant différente de la deuxième distance inter-électrode.

De préférence, la distance inter-électrodes séparant la paire d'électrodes de travail est choisie suffisamment petite pour que les changements de la réponse physiologique du sujet soient négligeables pendant la durée du retard temporel entre les variations des signaux ampérométriques des électrodes de travail, et suffisamment grande pour permettre, au moins dans l'un des canaux microfluidiques, un régime de fonctionnement découplé des électrodes de travail.

En effet, le régime de fonctionnement - couplé ou découplé - des électrodes de travail est fonction de la vitesse d'écoulement moyenne du fluide dans le canal microfluidique et de la distance inter-électrodes. Aux vitesses d'écoulement élevées, si la distance inter-électrodes est trop faible, le flux de fluide qui a réagi à la première électrode de travail est encore inhomogène après avoir atteint la deuxième électrode de travail. Ce régime de couplage limite la résolution temporelle des signaux ampérométriques, ce qui dégrade la précision des mesures de débit volumique de sueur.

Selon un mode de réalisation, le système de mesure électrochimique par ampérométrie est configuré pour déterminer le débit volumique en fonction d'une surface de section dudit canal microfluidique selon le sens d'écoulement.

En configurant la distance inter-électrode de manière différente selon la cellule électrochimique considérée, il est ainsi possible de mesurer un débit volumique sur une plage de valeurs couvrant l'ensemble des débits physiologiques envisageables.

Selon un mode de réalisation, l'invention fournit un appareil destiné à être posé sur une zone d'investigation d'un épiderme d'un sujet humain ou animal pour mesurer un paramètre quantitatif de sudation du sujet, ledit appareil comportant :

- une structure définissant un dispositif électrochimique microfluidique, la structure comportant un orifice d'entrée définissant la zone d'investigation et laissant passer une sueur depuis l'épiderme, l'au moins un canal microfluidique du dispositif électrochimique microfluidique étant en communication avec l'orifice d'entrée ; et
- un dispositif électronique de traitement configuré pour déterminer le paramètre quantitatif de sudation dudit sujet humain ou animal à partir de mesures du débit volumique de sueur réalisées par le dispositif électrochimique microfluidique.

Le paramètre quantitatif de sudation peut être un taux de sudation déterminé à partir du volume total de sueur transpirée par le sujet sur une gamme de temps donnée, rapportée à la surface de la zone d'investigation.

Selon un mode de réalisation, le paramètre quantitatif de sudation dudit sujet humain ou animal est un taux de sudation.

On entend par épiderme la couche superficielle de la peau chez l'homme et les animaux.

Selon des modes de réalisation, un tel appareil peut comporter une ou plusieurs des caractéristiques suivantes.

Selon un mode de réalisation, la structure est une structure multicouche comportant une couche inférieure et au moins une couche superposée sur la couche inférieure, le dispositif électrochimique microfluidique s'étendant parallèlement à la couche inférieure, la couche inférieure comprenant ledit orifice d'entrée.

Selon un mode de réalisation, la structure multicouche comporte en outre une couche supérieure et au moins une couche intermédiaire située entre la couche inférieure et la couche supérieure, le dispositif électrochimique microfluidique étant formé dans l'épaisseur de l'au moins une couche intermédiaire.

Les couches peuvent être fixées les unes aux autres par toute méthode appropriée, par exemple par des adhésifs, par soudage, par serrage mécanique etc.

Grâce à ces caractéristiques, la fabrication, le montage et donc l'industrialisation de l'appareil est facilité.

Grâce à ces caractéristiques, l'appareil est adapté aux éventuelles courbures lorsqu'il est appliqué sur l'épiderme. En outre, la ou les couches intermédiaires permettent également de créer une épaisseur permettant de compenser l'épaisseur des électrodes du dispositif électrochimique microfluidique. Ainsi, l'étanchéité de l'appareil est assurée.

Selon un mode de réalisation, la couche supérieure comporte un orifice de sortie traversant la couche supérieure, et dans lequel l'au moins un canal microfluidique est en communication avec l'orifice de sortie.

Selon un mode de réalisation, la première électrode de travail, l'au moins une deuxième électrode de travail, ladite au moins une deuxième électrode de travail, l'au moins une contre-électrode et l'au moins une électrode de référence sont disposées sur une face interne de la couche supérieure fermant l'au moins un canal microfluidique par le haut et/ou sur une face supérieure de la couche inférieure fermant ledit au moins un canal microfluidique par le bas.

Grâce à ces caractéristiques, les électrodes sont disposées de manière fiable. En outre, la fabrication de la structure multicouche comportant ces électrodes est facilitée en ce qu'il est possible de fabriquer les électrodes sur une couche plane lorsque le dispositif électrochimique microfluidique est formé dans une couche intermédiaire.

Selon un mode de réalisation, l'appareil comporte en outre un dispositif de communication, filaire ou sans fil, configuré pour transmettre un ou plusieurs signaux de mesure produits par le dispositif électrochimique microfluidique.

Selon un mode de réalisation, l'appareil comporte en outre un module gyroscopique et/ou au moins un accéléromètre pour détecter un état d'activité dudit sujet humain ou animal.

Selon un mode de réalisation, l'appareil comporte en outre un capteur de température configuré pour mesurer la température de l'épiderme dudit sujet humain ou animal.

Selon un mode de réalisation, l'appareil comprend un module de géolocalisation.

Grâce à ces caractéristiques, l'appareil est configuré pour effectuer et transmettre périodiquement des mesures, par exemple à une fréquence paramétrable ou à une fréquence dépendant d'un état d'activité détecté par l'appareil afin de faciliter une analyse des corrélations entre l'état d'activité du sujet et le paramètre quantitatif de sudation mesuré par l'appareil.

Les mesures du débit volumique de sueur et/ou du paramètre quantitatif de sudation peuvent être exploitées dans diverses applications, par exemple pour suivre le niveau d'hydratation du sujet afin de prévenir les déséquilibres hydriques corporels, notamment chez les sportifs pendant un effort ou chez les personnes âgées, particulièrement en cas de fortes chaleurs ou encore diagnostiquer l'hypohidrose, quelles qu'en soient les causes.

D'autres applications sont possibles dans divers domaines technologiques ou environnementaux dès lors que la mesure d'un débit est nécessaire dans un dispositif ou un procédé impliquant un fluide électroactif ou contenant une ou plusieurs espèces électroactives.

**Brève description des figures**

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.

[Fig.1] La [Fig.1] est une vue schématique d'un sujet vu de dos sur lequel a été mis en place un appareil selon un mode de réalisation.

[Fig.2] La [Fig.2] est une vue en perspective représentant partiellement une structure multicouche pour un appareil selon un mode de réalisation.

[Fig.3] La [Fig.3] est une vue en coupe selon la ligne III-III de la [Fig.2].

[Fig.4] La [Fig.4] est une vue éclatée de la structure multicouche selon un mode de réalisation.

[Fig.5] La [Fig.5] est une représentation schématique fonctionnelle partielle d'une structure multicouche définissant un dispositif électrochimique dans un appareil.

[Fig.6] La [Fig.6] est une représentation schématique fonctionnelle partielle d'un dispositif électrochimique microfluidique pouvant être utilisé dans un appareil.

[Fig.7] La [Fig.7] est une vue schématique du dessus d'une cellule électrochimique selon un premier mode de réalisation.

[Fig.8] La [Fig.8] est une vue schématique analogue à celle de la [Fig.7], selon un deuxième mode de réalisation.

[Fig.9] La [Fig.9] est une représentation schématique fonctionnelle en coupe d'une cellule électrochimique le long d'un canal microfluidique selon un premier mode de réalisation.

[Fig.10] La [Fig.10] est un ensemble de chronoampérogrammes illustrant un procédé pouvant être mise en œuvre, selon le premier mode de réalisation, avec le dispositif électrochimique microfluidique de la [Fig.7].

[Fig.11] La [Fig.11] est une représentation schématique fonctionnelle analogue à celle de la [Fig.9] selon un deuxième mode de réalisation.

[Fig.12] La [Fig.12] est un ensemble de chronoampérogrammes analogues à ceux de la [Fig.10] selon le deuxième mode de réalisation.

[Fig.13] La [Fig.13] est une représentation schématique fonctionnelle analogue à celles des figures 9 et 11, selon un troisième mode de réalisation.

[Fig.14] La [Fig.14] est un ensemble de chronoampérogrammes analogues à ceux des figures 10 et 12, selon un troisième mode de réalisation.

[Fig.15] La [Fig.15] est la représentation schématique fonctionnelle d'un dispositif électronique de contrôle pouvant être mis en œuvre avec l'appareil.

## Description des modes de réalisation

Les modes de réalisation ci-après décrits sont en relation avec un appareil pour déterminer un paramètre quantitatif de sudation d'un sujet au moyen d'un dispositif électrochimique microfluidique de mesure en continu du débit volumique de sueur dans un canal microfluidique. Plus généralement, un tel dispositif électrochimique microfluidique peut être incorporé dans de nombreux microsystèmes pour mesurer *in situ* la vitesse moyenne d'écoulement d'un fluide électroactif dans un canal microfluidique. Ces microsystèmes peuvent être par exemple des plateformes microfluidiques de type laboratoires-sur-puce *(lab-on-a-chip)* ou des microsystèmes d'analyse totale ( *micro-Total Analysis System* ou μTAS).

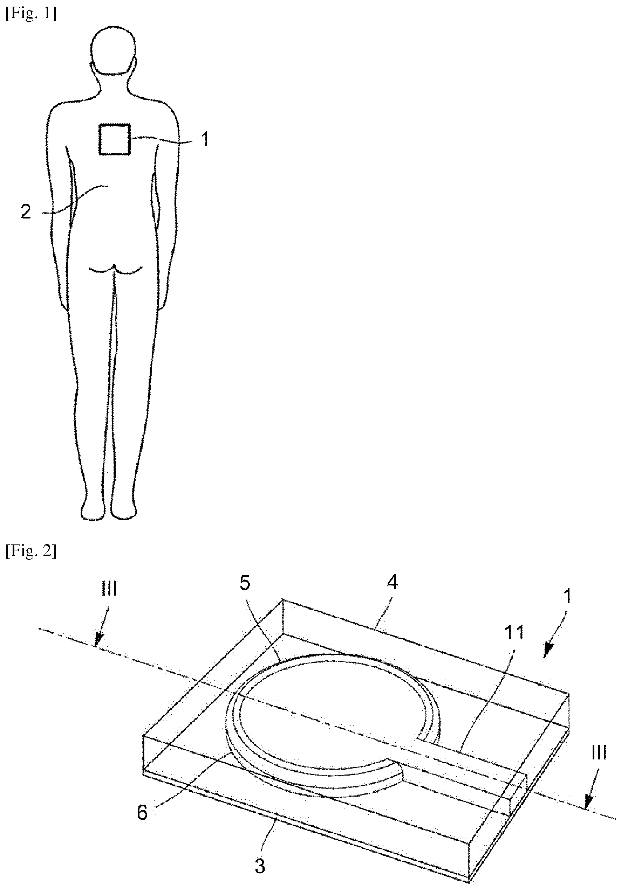

En référence à la [Fig.1], l'appareil 1 pour déterminer un paramètre quantitatif de sudation est disposé sur la peau 2 d'un sujet humain, par exemple dans son dos. Dans une variante non représentée, l'appareil 1 peut être disposée sur la peau d'un sujet animal.

En référence à la [Fig.2], l'appareil 1 se présente par exemple sous la forme d'une structure multicouche peu encombrante, faite de matériaux étanches à l'eau, par exemple en matériau polymère. La structure multicouche comporte une couche inférieure 3 faite d'un matériau souple et biocompatible, de préférence autocollant, par exemple en polytéréphtalate d'éthylène (PET), que l'on peut positionner directement sur la peau 2 du sujet et un support isolant 4 superposé à la couche inférieure 3.

Un canal microfluidique 11 est creusé dans l'épaisseur du support isolant 4. Une cupule de prélèvement 5, située au droit d'une ouverture circulaire 6, est pratiquée dans la couche inférieure 3.

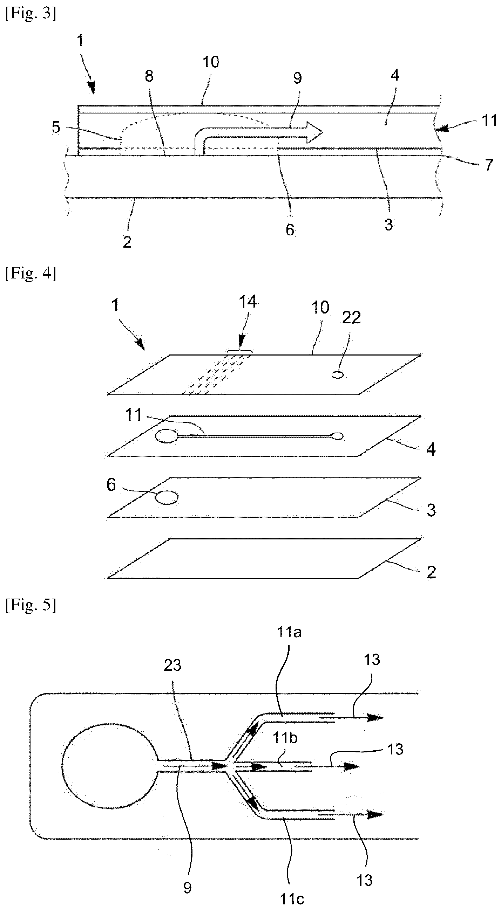

En référence à la [Fig.3], la couche inférieure 3 est collée sur la peau 2 par une couche adhésive 7. Une partie centrale de la couche inférieure 3 et de la couche adhésive 7 comporte l'ouverture circulaire 6 délimitant une zone d'investigation 8 sur la peau 2 du sujet, faisant par exemple quelques millimètres à quelques centimètres de diamètre. L'ouverture circulaire 6 peut prendre une autre forme, par exemple une ellipse, un triangle, un rectangle, un carré, un polygone ou autre. L'ouverture circulaire 6 forme un orifice d'entrée 6 permettant de guider un flux de sueur 9, notamment pour amener la sueur dans le canal microfluidique 11. Le flux de sueur 9 passe depuis la peau 2 du sujet jusque dans le canal microfluidique 11 en passant à travers l'ouverture circulaire 6.

Un élément collecteur hydrophile (non représenté), par exemple un corps fibreux tel que du coton ou un matériau non tissé peut être disposé dans l'ouverture circulaire 6 et la cupule de prélèvement 5. L'élément collecteur remplit la fonction d'amener la sueur produite dans la zone d'investigation 8 jusqu'au dispositif électrochimique microfluidique 100.

Selon un premier mode de réalisation, en référence à la [Fig.4], la structure multicouche de l'appareil 1 comporte une couche inférieure 3 comprenant un orifice d'entrée 6 laissant passer la sueur, une couche supérieure 10 comprenant un orifice de sortie 22, une couche intermédiaire 4 située entre la couche inférieure 3 et la couche supérieure 10, le dispositif électrochimique microfluidique 100 étant formé dans l'épaisseur du support isolant 4 constituant une couche intermédiaire 4 s'étendant parallèlement à la couche inférieure 3.

Le dispositif électrochimique microfluidique 100 comporte un canal microfluidique 11 qui est en communication avec l'orifice d'entrée 6 en une première extrémité et en communication avec l'orifice de sortie 22 en deuxième extrémité. Ainsi, le flux de sueur 9 provenant de la peau 2 du sujet est amené dans le canal microfluidique 11 qui guide la sueur depuis l'orifice d'entrée 6 jusqu'à l'orifice de sortie 22 par capillarité.

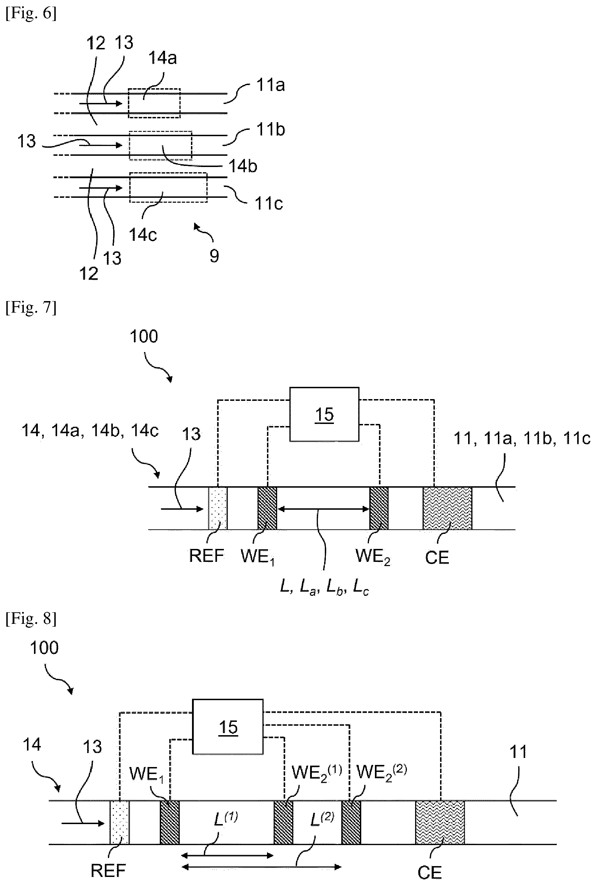

Le canal microfluidique 11 est muni d'une cellule électrochimique 14 ci-après décrite, représentée en [Fig.7] ou en [Fig.8]. La cellule électrochimique 14 est disposée sur la face interne de la couche supérieure 10 fermant le canal microfluidique 11 par le haut de sorte à être située dans l'espace interne du canal microfluidique 11.

En dimensionnement, l'orifice d'entrée 6 présente un diamètre de quelques millimètres, le canal microfluidique 11 présente une longueur comprise entre 0,5 cm et 5 cm et une largeur comprise entre 20 $\mu$m et 1000 $\mu$m, la couche intermédiaire 4 présente une épaisseur comprise entre 10 $\mu$m et 500 $\mu$m, les couches 3, 4, 10 de la structure multicouche présentent une largeur comprise entre 1 cm et 5 cm et une longueur comprise entre 2 cm et 10 cm.

Par exemple, l'orifice d'entrée 6 présente un diamètre de 5 mm, le canal microfluidique 11 présente une longueur de 3 cm et d'une largeur de 200 $\mu$m, la couche intermédiaire 4 présente une épaisseur de 150 $\mu$m, les couches 3, 4, 10 de la structure multicouche présentent une largeur de 3 cm et une longueur de 9 cm.

Selon un deuxième mode de réalisation, en référence aux figures 5 et 6, l'appareil 1 comporte un canal principal 23 se divisant dans le sens de l'écoulement du flux de sueur 9 en un ou plusieurs canaux microfluidiques, ici trois canaux microfluidiques 11a, 11b, 11c parallélépipédiques, parallèles entre eux, formés dans l'épaisseur du support isolant 6 et séparés par des cloisons 12. Chaque canal microfluidique 11a, 11b, 11c est ainsi respectivement séparé des autres canaux microfluidiques 11a, 11b, 11c au sein desquels la sueur peut circuler de manière indépendante. Le nombre de canaux microfluidiques peut être plus élevé ou plus faible que celui représenté sur les figures 5 et 6.

En dimensionnement, les canaux microfluidiques 11a, 11b, 11c ont de préférence une hauteur comprise entre 10 $\mu$m et 500 $\mu$m, une largeur comprise entre 20 $\mu$m et 1000 $\mu$m, et une longueur comprise entre 0,5 cm et 5 cm. Les canaux microfluidiques 11a, 11b, 11c ont une section constante de surface $S_a$, $S_b$, $S_c$. Pour simplifier, mais sans perte de généralité, les sections des canaux microfluidiques 11a, 11b, 11c sont supposées présenter une même surface S, i.e. que $S_a = S_b = S_c = S$.

La sueur transpirée par le sujet 2 dans la zone d'investigation 8 est collectée par la partie de l'élément collecteur en contact avec la peau 2 du sujet puis transférée par capillarité jusqu'au canal principal 8 afin de circuler de manière indépendante dans les canaux microfluidiques 11a, 11b, 11c du dispositif électrochimique microfluidique 100. Les flèches 13 illustrent le sens d'écoulement de la sueur dans les canaux microfluidiques 11a, 11b, 11c. Chaque canal microfluidique 11a, 11b, 11c est respectivement muni d'une cellule électrochimique 14a, 14b, 14c indiquée en [Fig.6] et détaillée en [Fig.7] ou en [Fig.8]. De préférence, les canaux microfluidiques 11a, 11b, 11c aboutissent dans un réservoir de sortie (non représenté) en communication avec l'orifice de sortie 22. Le réservoir de sortie retient la sueur pour éviter de la remettre en contact avec la peau 2.

En référence à la [Fig.7], la cellule électrochimique 14, 14a, 14b, 14c repose sur une configuration à quatre électrodes. Plus précisément, la cellule électrochimique 14, 14a, 14b, 14c comporte une paire d'électrodes de travail $WE_1$ et $WE_2$ indépendantes, une contre-électrode CE et une électrode de référence REF, disposées dans le canal microfluidique 11, 11a, 11b, 11c. Les électrodes $WE_1$, $WE_2$, CE et REF sont réalisées sous la forme de bandes de microélectrodes parallèles entre elles, perpendiculaires à la direction d'écoulement de la sueur dans le canal microfluidique 11, 11a, 11b, 11c, et implantées par microfabrication, par exemple par dépôt chimique en phase vapeur (C *hemical Vapor Deposition* ou CVD) et/ou par lithographie. La longueur des bandes de microélectrodes correspond ainsi à la largeur du canal microfluidique 11, 11a, 11b, 11c.

Les électrodes de travail $WE_1$ et $WE_2$ peuvent être réalisées sous la forme de microbandes de platine ou de platine platiné - aussi appelé noir de platine - d'épaisseur nanométrique, par exemple de l'ordre de quelques dizaines de nanomètres à une centaine de nanomètres, typiquement 200 nm. Les électrodes de travail $WE_1$ et $WE_2$ sont espacées d'une distance inter-électrodes $L$, $L_a$, $L_b$, $L_c$ dans le sens d'écoulement de la sueur dans le canal microfluidique 11, 11a, 11b, 11c. Ainsi qu'il sera expliqué plus loin, la distance inter-électrodes $L$, $L_a$, $L_b$, $L_c$ varie en fonction du canal microfluidique 11, 11a, 11b, 11c considéré. On désignera par la première électrode de travail par la référence $WE_1$ et la deuxième électrode de travail par la référence $WE_2$. Par convention, la première électrode de travail $WE_1$ est située en amont de la deuxième électrode de travail $WE_2$ par rapport au sens d'écoulement de la sueur du sujet 2 dans le canal microfluidique 11, 11a, 11b, 11c.

L'électrode de référence REF, réalisée par exemple sous la forme d'une bande de mi-croélectrode de référence d'Ag/AgCl d'épaisseur nanométrique, par exemple 500 nm, est située en amont de la paire d'électrodes de travail $WE_1$ et $WE_2$ afin de préserver la stabilité du potentiel d'électrode de référence au cours du temps.

La contre-électrode CE, réalisée par exemple sous la forme d'une microbande par exemple en platine, platiné ou non, d'épaisseur nanométrique, par exemple de l'ordre de quelques dizaines de nanomètres, à quelques centaines de nanomètres, typiquement 100 nm, est située en aval de la paire d'électrodes de travail $WE_1$ et $WE_2$ et, partant, de l'électrode de référence REF de sorte que les espèces chimiques générées à sa surface ne perturbent ni les électrodes de travail $WE_1$ et $WE_2$ ni l'électrode de référence REF. Avantageusement, la surface de la contre-électrode CE est deux à trois fois plus grande que celles des autres électrodes.

Avantageusement, les microélectrodes réalisées sous la forme de microbandes sont toutes déposées sur une couche d'accroche sub-nanométrique (non représentée), par exemple en titane ou en chrome ou autre selon la nature du support isolant 4, pour procurer une bonne adhésion des microbandes sur le support isolant 4.

Le dispositif électrochimique microfluidique 100 comporte également un système de mesure électrochimique par ampérométrie 15. Chacune des électrodes $WE_1$, $WE_2$, CE et REF est connectée au système de mesure électrochimique par ampérométrie 15 par l'intermédiaire de contacts électriques (non représentés) isolés électriquement de la sueur du sujet.

Le système de mesure électrochimique par ampérométrie 15 comporte par exemple un potentiostat ou un multipotentiostat (non représenté) configuré pour commander une des ou toutes les ou certaines des cellules électrochimiques 14, 14a, 14b et 14c. Plus précisément, le système de mesure électrochimique par ampérométrie 15 est configuré pour polariser les première et deuxième électrodes de travail $WE_1$ et $WE_2$ d'une cellule électrochimique 14, 14a, 14b, 14c respectivement aux premier et deuxième potentiels d'électrode E1 et E2 de sorte à générer dans la sueur une réaction d'oxydation ou une réaction de réduction associée à l'eau $H_2O_{(1)}$ (exemples 1 et 2) ou encore la réaction d'une espèce chimique d'un couple rédox associé à l'eau $H_2O_{(1)}$, en particulier le dioxygène $O_{2(aq)}$ préalablement dissous dans la sueur (exemple 3).

Le débit volumique Q moyen de sueur circulant dans un canal microfluidique 11, 11a, 11b ou 11c est déterminé en se fondant sur le principe de la technique dite du « temps de vol » (en anglais : « *time of flight* »), c'est-à-dire à partir de la mesure du temps nécessaire pour une espèce chimique électroactive, détectée par ampérométrie, pour parcourir la distance inter-électrodes $L$, $L_a$, $L_b$, $L_c$ comprise entre les première et deuxième électrodes de travail $WE_1$ et $WE_2$, et du volume du canal microfluidique 11, 11a, 11b, 11c délimité par les plans perpendiculaires au plan dans lequel sont serties les électrodes de travail $WE_1$, $WE_2$ se situant à la limite la plus amont de chacune.

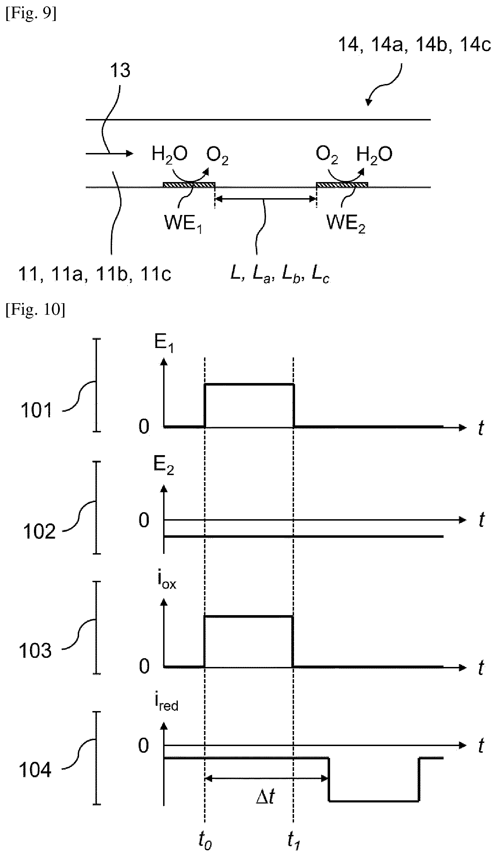

Dans les trois exemples qui suivent, les électrodes de travail $WE_1$ et $WE_2$ sont respectivement polarisées selon les procédés illustrés aux figures 9 et 10 (exemple 1), 11 et 12 (exemple 2), et 13 et 14 (exemple 3). Ces procédés comportent plusieurs étapes expliquées plus en détail pour chacun des exemples particuliers ci-après décrits. Les graphiques 101, 121 et 141 représentent le premier potentiel d'électrode $E_1$ appliqué à la première électrode de travail $WE_1$ en fonction du temps $t$. Les graphiques 102, 122 et 142 représentent le deuxième potentiel d'électrode $E_2$ appliqué à la deuxième électrode de travail $WE_2$ en fonction du temps $t$. Lorsque les premier et deuxième potentiels d'électrode $E_1$ et $E_2$ prennent la valeur « 0 » sur les graphiques 101, 102, 121, 122, 141 et 142, cela correspond à la déconnexion de l'électrode de travail $WE_1$ ou $WE_2$ correspondante (circuit ouvert) ou bien à l'application d'un potentiel proche ou égal au potentiel d'équilibre. Les graphiques 103, 123 et 143 représentent l'intensité du courant Faradique mesurée à la première électrode de travail $WE_1$ en fonction du temps t. Enfin, les graphiques 104, 124 et 144 représentent l'intensité du courant Faradique mesurée à la deuxième électrode de travail $WE_2$ en fonction du temps t.

Les valeurs des potentiels d'électrode $E_1$ et $E_2$ indiquées à titre d'exemple sont données en volt par rapport l'électrode standard à hydrogène (V/ESH). Par convention, l'intensité anodique du courant Faradique emprunte des valeurs positives, tandis que l'intensité cathodique du courant Faradique emprunte des valeurs négatives.

*Exemple 1*

Dans un premier exemple, en référence à la [Fig.9], la différence de potentiels d'électrode entre la première électrode de travail $WE_1$ et la contre-électrode CE est fixée de sorte que la première électrode de travail $WE_1$ est polarisée à un premier potentiel d'électrode $E_1$, typiquement 1,6 V/ESH, permettant d'oxyder l'eau $H_2O_{(1)}$ en dioxygène $O_{2(aq)}$ selon la demi-équation d'oxydoréduction :

$$6 \ H_2O_{(1)} \rightarrow O_{2(aq)} + 4 \ H_3O^+_{(aq)} + 4 \ e$$

Corrélativement, la différence de potentiels d'électrode entre la deuxième électrode de travail $WE_2$ et la contre-électrode CE est fixée de sorte que la deuxième électrode de travail $WE_2$ est polarisée à un deuxième potentiel d'électrode $E_2$, typiquement - 0,3 V/ESH, permettant de réduire l'oxygène $O_{2(aq)}$ produit à la surface de l'électrode de travail $WE_1$ dissous dans la sueur en eau $H_2O_{(1)}$ selon la demi-équation d'oxydoréduction :

$$O_{2(aq)} + 4 \ H_3O^+_{(aq)} + 4 \ e \rightarrow 6 \ H_2O_{(1)}$$

En référence à la [Fig.10], le procédé de polarisation des première et deuxième électrodes de travail $WE_1$ et $WE_2$ comportent deux étapes successives dans le temps $t$.

Pendant une première étape, avant un instant $t_0$ (i.e. pour $t < t_0$) le premier potentiel d'électrode $E_1$ appliqué à la première électrode de travail $WE_1$ peut être proche du potentiel d'équilibre initial ou bien la première électrode de travail $WE_1$ est déconnectée. Dans cette dernière hypothèse, illustrée sur le graphique 101, le premier potentiel d'électrode $E_1$ emprunte par convention la valeur nulle « 0 ». L'intensité anodique $i_{ox}$ détectée est nulle, ce qu'indique le graphique 103.

Parallèlement, le deuxième potentiel d'électrode $E_2$ appliqué à la deuxième électrode de travail $WE_2$, illustré sur le graphique 102, est imposé à une valeur inférieure au potentiel d'équilibre initial et suffisante pour réduire le dioxygène

$O_{2(aq)}$ dissous. L'intensité cathodique $i_{red}$ est proportionnelle à la concentration en dioxygène $O_{2(aq)}$ préalablement dissous dans la sueur, ce qu'illustre le graphique 104 ; si cette concentration initiale est nulle, l'intensité cathodique $i_{red}$ est nulle.

Pendant une deuxième étape débutant à partir de l'instant $t_0$, le premier potentiel d'électrode $E_1$ appliqué à la première électrode de travail $WE_1$, illustré sur le graphique 101, est fixé sur la vague d'oxydation de l'eau $H_2O_{(1)}$ de sorte à initier la production de dioxygène $O_{2(aq)}$ en quantité appréciable, autrement dit de sorte que la concentration totale en dioxygène $O_{2(aq)}$ au voisinage de la surface de la première électrode de travail $WE_1$ est très supérieure à la concentration en dioxygène $O_{2(aq)}$ préalablement dissous dans la sueur. L'intensité anodique $i_{ox}$, grandeur représentative de la quantité de dioxygène $O_{2(aq)}$ généré à la surface de la première électrode de travail $WE_1$, est ainsi fixée par le premier potentiel d'électrode $E_1$ appliqué à la première électrode de travail $WE_1$. L'intensité anodique $i_{ox}$ est constante dans le temps dès que le courant capacitif lié au saut de potentiel s'annule car la réaction rédox envisagée à la première électrode de travail $WE_1$ n'est pas limitée par le transport de matière puisque le réactif est l'eau $H_2O_{(1)}$. Les courants capacitifs liés aux commutation de potentiel ne sont pas représentés sur les schémas qui ne considèrent que les courants Faradiques.

Dès la polarisation de la première électrode de travail $WE_1$ à l'instant $t_0$, un gradient en concentration de dioxygène $O_{2(aq)}$ dissous est créé au voisinage de la première électrode de travail $WE_1$. L'intensité anodique $i_{ox}$ mesurée à la première électrode de travail $WE_1$ augmente du fait de l'oxydation de l'eau $H_2O_{(1)}$. Sur le graphique 103, la croissance de l'intensité anodique $i_{ox}$ est schématisée par un échelon ou une fonction de Heaviside. Le gradient de concentration en dioxygène $O_{2(aq)}$ forme un front de concentration entraîné par convection en aval de la première électrode de travail $WE_1$ sous l'effet de l'écoulement de la sueur.

Parallèlement, la deuxième électrode de travail $WE_2$ demeure polarisée au deuxième potentiel d'électrode $E_2$ constant. La deuxième électrode de travail $WE_2$ enregistre en continu l'intensité cathodique $i_{red}$ du courant Faradique généré par la réduction du dioxygène $O_{2(aq)}$ en eau $H_2O_{(1)}$. Ainsi, à l'instant $t_0 + \Delta t$, lorsque le front de concentration en dioxygène $O_{2(aq)}$ généré à la surface de la première électrode de travail $WE_1$ passe au-dessus de la surface de la deuxième électrode de travail $WE_2$, l'intensité cathodique $i_{red}$ détectée diminue (en valeur relative) du fait de la réduction du dioxygène $O_{2(aq)}$ en eau $H_2O_{(1)}$, ce qu'indique le graphique 104. La durée $\Delta t$ correspond au temps nécessaire pour que le front de dioxygène $O_{2(aq)}$ généré à la première électrode de travail $WE_1$ transite jusqu'à la deuxième électrode de travail $WE_2$ sous l'effet de l'écoulement de la sueur dans le canal microfluidique 11, 11a, 11b ou11c à la vitesse d'écoulement V.

La deuxième étape se termine à un instant $t_1$ postérieur à l'instant $t_0$, à partir duquel la première électrode de travail $WE_1$ est à nouveau polarisée à un premier potentiel d'électrode $E_1$ proche du potentiel d'équilibre initiale, ou bien est déconnectée ainsi que cela est illustré sur le graphique 101. La première électrode de travail $WE_1$ peut être ultérieurement repolarisée de sorte que le procédé peut être réitéré autant que de nécessaire pour déterminer le débit volumique moyen Q à des instants successifs plus ou moins rapprochés.

La méthode décrite ici est simple et facilement industrialisable, car sans pièce mobile ni aucune hypothèse sur le régime hydrodynamique de l'écoulement de la sueur dans le canal microfluidique. La solution n'est aucunement liée à la détermination de concentration d'espèces chimiques générées ou contenues dans la sueur, mais uniquement à un délai de réponse $\Delta t$ entre les signaux ampérométriques une paire d'électrodes de travail $WE_1$ et $WE_2$. En particulier, le choix des réactions d'oxydation de l'eau $H_2O_{(1)}$ et de réduction du dioxygène $O_{2(aq)}$ permet de piloter l'amplitude des signaux ampérométriques détectés à chacune des électrodes de travail $WE_1$, $WE_2$ de sorte à maintenir un rapport signal sur bruit suffisant pour permettre une détection aisée de la variation des signaux ampérométriques.

*Exemple 2*

Dans un deuxième exemple, en référence à la [Fig.1]1, la différence de potentiels d'électrode entre la première électrode de travail $WE_1$ et la contre-électrode CE est fixée de sorte que la première électrode de travail $WE_1$ est polarisée à un premier potentiel d'électrode $E_1$, typiquement -0,8 V/ESH, permettant de réduire l'eau $H_2O_{(1)}$ en dihydrogène $H_{2(aq)}$ selon la demi-équation d'oxydoréduction

$$2\ H_2O_{(1)} + 2\ e \rightarrow H_{2(aq)} + 2\ OH_{(aq)}^-$$

Corrélativement, la différence de potentiels d'électrode entre la deuxième électrode de travail $WE_2$ et la contre-électrode CE est fixée de sorte que la deuxième électrode de travail $WE_2$ est polarisée à un deuxième potentiel d'électrode $E_2$ égal au premier potentiel d'électrode $E_1$. À l'instar de la première électrode de travail $WE_1$, la deuxième électrode de travail $WE_2$ est ainsi configurée pour réduire l'eau $H_2O_{(1)}$ de la sueur en dihydrogène $H_{2(aq)}$.

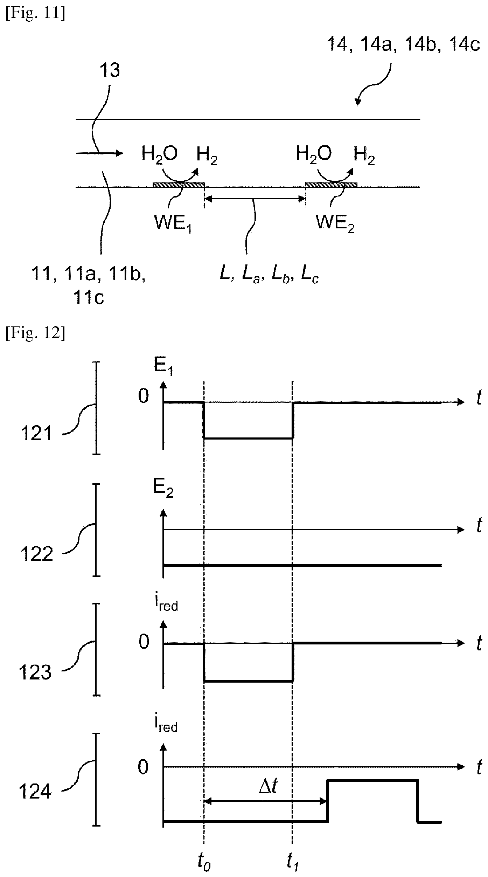

En référence à la [Fig.12], le procédé de polarisation des première et deuxième électrodes de travail $WE_1$ et WE2 comportent deux étapes successives dans le temps t.

Pendant une première étape, avant un instant $t_0$, i.e. pour $t < t_0$, le premier potentiel d'électrode $E_1$ appliqué à la première électrode de travail $WE_1$ peut être proche du potentiel d'équilibre initial ou bien la première électrode de travail $WE_1$ est déconnectée. Dans cette dernière hypothèse, illustrée sur le graphique 121, le premier potentiel d'électrode $E_1$

emprunte par convention la valeur nulle « 0 », de sorte que l'intensité cathodique $i_{red}$ détectée est nulle, ce qu'indique le graphique 123.

Parallèlement, le deuxième potentiel d'électrode $E_2$ appliqué à la deuxième électrode de travail $WE_2$, illustré sur le graphique 112, est inférieur au potentiel d'équilibre initial pour réduire l'eau de la sueur en dihydrogène $H_{2(aq)}$. L'intensité cathodique $i_{red}$ est constante, ce qu'illustre le graphique 124.

Pendant une deuxième étape débutant à partir de l'instant $t_0$, le premier potentiel d'électrode $E_1$ appliqué à la première électrode de travail $WE_1$, illustré sur le graphique 111, est plus faible que le potentiel d'équilibre initial de sorte à initier la réduction de l'eau de la sueur en dihydrogène $H_{2(aq)}$. L'intensité cathodique $i_{red}$ détectée à la première électrode de travail $WE_1$ diminue (en valeur relative) du fait de l'augmentation du pH imposée par la réduction de l'eau $H_2O_{(1)}$ à la première électrode de travail $WE_1$. Sur le graphique 123, la diminution de croissance de l'intensité cathodique $i_{red}$ est schématisée par un échelon ou une fonction de Heaviside. La portion de la sueur hydrolysée est entraînée par convection en aval de la première électrode de travail $WE_1$ sous l'effet de l'écoulement.

Parallèlement, la deuxième électrode de travail $WE_2$ demeure polarisée au deuxième potentiel d'électrode $E_2$ constant. La deuxième électrode de travail $WE_2$ enregistre en continu l'intensité cathodique $i_{red}$ du courant faradique généré par la réduction de l'eau de la sueur en dihydrogène $H_{2(aq)}$. Ainsi, à l'instant $t_0 + \varDelta t$, lorsque le flux de sueur partiellement hydrolysé passe au-dessus de la surface de la deuxième électrode de travail $WE_2$, l'intensité cathodique $i_{red}$ détectée augmente (en valeur relative), i.e. puisque la concentration en ions hydroniums $H_3O^+_{(aq)}$ est plus faible qu'en amont de la première électrode de travail $WE_1$, ce qu'indique le graphique 114. La durée $\varDelta t$ correspond au temps nécessaire pour que le front de flux de sueur déplété en ions hydroniums $H_3O^+_{(aq)}$ généré à la première électrode de travail $WE_1$ transite jusqu'à la deuxième électrode de travail $WE_2$ sous l'effet de l'écoulement de la sueur dans le canal microfluidique 11, 11a, 11b, 11c à la vitesse d'écoulement V.

Le volume de sueur dont le pH a été augmenté par l'action de la première électrode transite de la première électrode de travail $WE_1$ jusqu'à la deuxième électrode de travail $WE_2$ sous l'effet de l'écoulement de la sueur dans le canal microfluidique 11, 11a, 11b, 11c à la vitesse d'écoulement V.

La deuxième étape se termine à un instant $t_1$ postérieur à l'instant $t_0$, à partir duquel la première électrode de travail $WE_1$ est à nouveau polarisée à un premier potentiel d'électrode $E_1$ proche du potentiel d'équilibre initial, ou bien est déconnectée ainsi que cela est illustré sur le graphique 121. La première électrode de travail $WE_1$ peut être ultérieurement repolarisée de sorte que le procédé peut être réitéré autant que de nécessaire pour déterminer le débit volumique moyen Q à des instants successifs rapprochés.

La méthode décrite ici est simple et facilement industrialisable, car sans pièce mobile ni aucune hypothèse sur le régime hydrodynamique. La solution n'est aucunement liée à la détermination de concentration d'espèces chimiques générées ou contenues dans la sueur, mais uniquement à un délai de réponse $\varDelta t$ entre les variations des signaux ampérométriques de la paire d'électrodes de travail $WE_1$ et $WE_2$.

*Exemple 3*

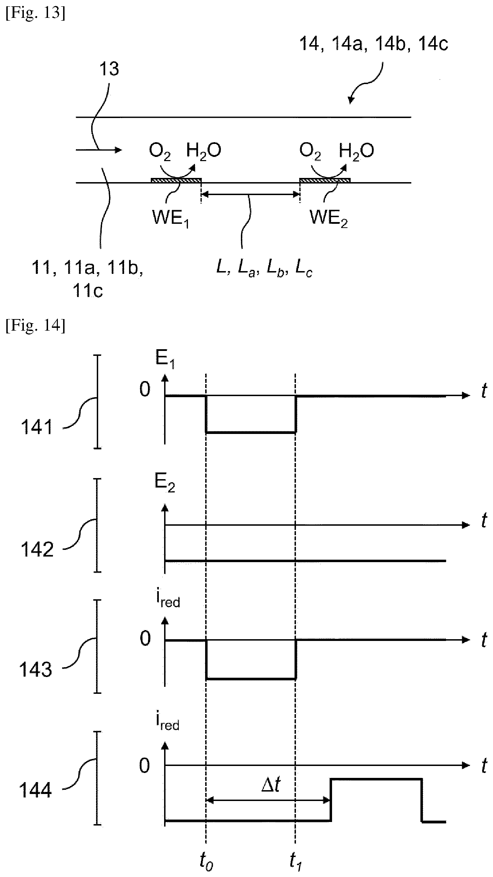

Dans un troisième exemple, en référence à la [Fig.13], la différence de potentiels d'électrode entre la première électrode de travail $WE_1$ et la contre-électrode CE est fixée de sorte que la première électrode de travail $WE_1$ est polarisée à un premier potentiel d'électrode $E_1$, typiquement -0,3 V/ESH, permettant de réduire uniquement l'oxygène $O_{2(aq)}$ initialement dissous dans la solution aqueuse examinée, lorsqu'elle en contient, en eau $H_2O_{(1)}$ selon la demi-équation d'oxydoréduction

$$O_{2(aq)} + 4\ H_3O^+_{(aq)} + 4\ e \rightarrow 6\ H_2O_{(1)}$$

Corrélativement, la différence de potentiels d'électrode entre la deuxième électrode de travail $WE_2$ et la contre-électrode CE est fixée de sorte que la deuxième électrode de travail $WE_2$ est polarisée à un deuxième potentiel d'électrode $E_2$ égale au premier potentiel d'électrode $E_1$. À l'instar de la première électrode de travail $WE_1$, la deuxième électrode de travail $WE_2$ est ainsi configurée pour réduire la fraction d'oxygène $O_{2(aq)}$ dissous dans la sueur en eau $H_2O_{(1)}$ qui n'a pas été réduite à la première électrode de travail $WE_1$.

En référence à la [Fig.14], le procédé de polarisation des première et deuxième électrodes de travail $WE_1$ et WE2 comportent deux étapes successives dans le temps t.

Pendant une première étape, avant un instant $t_0$, i.e. pour $t < t_0$, le premier potentiel d'électrode $E_1$ appliqué à la première électrode de travail $WE_1$ peut être proche du potentiel d'équilibre initial ou bien la première électrode de travail $WE_1$ est déconnectée. Dans cette dernière hypothèse, illustrée sur le graphique 141, le premier potentiel d'électrode $E_1$ emprunte par convention la valeur nulle « 0 », de sorte que l'intensité cathodique $i_{red}$ détectée est nulle, ce qu'indique le graphique 143.

Parallèlement, le deuxième potentiel d'électrode $E_2$ appliqué à la deuxième électrode de travail $WE_2$, illustré sur le graphique 142, est inférieur au potentiel d'équilibre initial pour réduire le dioxygène $O_{2(aq)}$ en eau $H_2O_{(1)}$. L'intensité

cathodique $i_{red}$ est constante puisque proportionnelle à la concentration en en dioxygène $O_{2(aq)}$ préalablement dissous dans la sueur, ce qu'illustre le graphique 144.

Pendant une deuxième étape débutant à partir de l'instant $t_0$, le premier potentiel d'électrode $E_1$ appliqué à la première électrode de travail $WE_1$, illustré sur le graphique 141, est plus faible que le potentiel d'équilibre initial de sorte à initier la réduction de tout ou partie du dioxygène $O_{2(aq)}$ dissous dans la sueur. L'intensité cathodique $i_{red}$ détectée à la première électrode de travail $WE_1$ diminue (en valeur relative) du fait de la réduction du dioxygène $O_{2(aq)}$ en eau $H_2O_{(1)}$. Sur le graphique 143, la diminution de croissance de l'intensité cathodique $i_{red}$ est schématisée par un échelon ou une fonction de Heaviside. La portion hydrolysée de la sueur déplétée en dioxygène $O_{2(aq)}$ dissous est entraînée par convection en aval de la première électrode de travail $WE_1$ sous l'effet de l'écoulement.

Parallèlement, la deuxième électrode de travail $WE_2$ demeure polarisée au deuxième potentiel d'électrode $E_2$ constant. La deuxième électrode de travail $WE_2$ enregistre en continu l'intensité cathodique $i_{red}$ du courant Faradique généré par la réduction du dioxygène $O_{2(aq)}$ en eau $H_2O_{(1)}$. Ainsi, à l'instant $t_0 + \Delta t$, lorsque le flux de sueur déplété en dioxygène $O_{2(aq)}$ passe au-dessus de la surface de la deuxième électrode de travail $WE_2$, l'intensité cathodique $i_{red}$ détectée augmente (en valeur relative), i.e. qu'elle se rapproche de la valeur nulle, puisque la concentration en dioxygène $O_{2(aq)}$ dissous dans la sueur est nulle ou, à tout le moins, plus faible qu'en amont de la première électrode de travail $WE_1$, ce qu'indique le graphique 144. La durée $\Delta t$ correspond au temps nécessaire pour que le flux de sueur déplété en dioxygène $O_{2(aq)}$ dissous transite de la première électrode de travail $WE_1$ jusqu'à la deuxième électrode de travail $WE_2$ sous l'effet de l'écoulement de la sueur dans le canal microfluidique 11, 11a, 11b, 11c à la vitesse d'écoulement $V$.

La deuxième étape se termine à un instant $t_1$ postérieur à l'instant $t_0$, à partir duquel la première électrode de travail $WE_1$ est à nouveau polarisée à un premier potentiel d'électrode $E_1$ proche du potentiel d'équilibre initial, ou bien est déconnectée ainsi que cela est illustré sur le graphique 141. La première électrode de travail $WE_1$ peut être ultérieurement repolarisée de sorte que le procédé peut être réitéré autant que de nécessaire pour déterminer le débit volumique moyen $Q$ à des instants successifs rapprochés.

La méthode décrite ici est simple et facilement industrialisable, car sans pièce mobile ni aucune hypothèse sur le régime hydrodynamique. La solution n'est aucunement liée à la détermination de concentration d'espèces chimiques générées ou contenues dans la sueur, mais uniquement à un délai de réponse $\Delta t$ entre les variations des signaux ampérométriques de la paire d'électrodes de travail $WE_1$ et $WE_2$.

Dans les trois exemples décrits ci-dessus, la vitesse d'écoulement $V$ et le débit volumique $Q$ de sueur circulant dans un canal microfluidique 11, 11a, 11b ou 11c de forme parallélépipédique rectangle présentant une section constante de surface S dans le sens d'écoulement 13 peuvent être déterminés à partir de la distance inter-électrodes $L$, $L_a$, $L_b$, $L_c$ séparant les électrodes de travail $WE_1$ et $WE_2$, et à partir de la durée $\Delta t$ caractéristique du délai de la réponse de la deuxième électrode de travail $WE_2$, suivie par chronoampérométrie, par rapport à la réponse instantanée de la première électrode de travail $WE_1$.

Sous les réserves qui seront exprimées ci-après, la vitesse linéaire d'écoulement moyenne $V$ et le débit volumique moyen $Q$ du flux de sueur circulant dans un canal microfluidique, par exemple le canal microfluidique référencé 11 et pour lequel la distance inter-électrode est référencée $L$, peuvent être estimées selon les équations suivantes :

$$V = L / \Delta t$$

$$Q = S \times V = L \times S / \Delta t$$

Sous les mêmes réserves, ces équations sont également valables, respectivement dans les canaux microfluidiques 11a, 11b, 11c en substituant la distance inter-électrodes $L$, par la distance inter-électrodes $L_a$, $L_b$, $L_c$.

Dans le cadre des applications dynamiques envisagées, pour le suivi temporel de l'état physiologique du sujet, il est souhaitable que l'appareil 1 pour déterminer le paramètre quantitatif de sudation d'un sujet mesure la valeur du débit volumique Q de sueur à des instants successifs rapprochés cohérents avec le rythme de sudation attendu, par exemple une fois par minute. L'intégration des variations temporelles du débit volumique Q de sueur permet ensuite de déterminer la valeur du flux de total de sueur transpirée par le sujet sur une gamme de temps t donné.

Le paramètre quantitatif de sudation peut être un taux de sudation déterminé à partir du volume total de sueur transpirée par le sujet sur une gamme de temps t donnée, rapportée à la surface de la zone d'investigation 8.

La distance inter-électrodes $L$, $L_a$, $L_b$, $L_c$ séparant les électrodes de travail $WE_1$ et $WE_2$ est choisie suffisamment petite pour que les changements de la réponse physiologique du sujet soient négligeables pendant la durée $\Delta t$ et suffisamment grande pour permettre un régime de fonctionnement découplé des électrodes de travail $WE_1$ et $WE_2$ dans le ou chaque canal microfluidique 11, 11a, 11b, 11c où la mesure de débit volumique Q est effectuée.

En effet, en fonction de la vitesse linéaire d'écoulement moyenne $V$ de la sueur dans le canal microfluidique 11, 11a, 11b ou 11c, le gradient de concentration créé au voisinage de la première électrode de travail, par génération d'espèces chimiques électroactives (exemple 1) ou par déplétion d'espèces chimiques électroactives déjà présentes dans la sueur (exemples 2 et 3), peut devenir ou non homogène sur la hauteur du canal microfluidique 11, 11a, 11b ou 11c après avoir été entraîné sur la distance inter-électrodes $L, L_a, L_b, L_c$. En particulier, lorsque compte tenu de la vitesse linéaire d'écoulement $V$ de la sueur, le gradient de concentration n'a pas le temps de se dissiper sur la hauteur du canal microfluidique 11a, 11b ou 11c avant d'atteindre la deuxième électrode de travail $WE_2$, le fonctionnement des deux électrodes de travail $WE_1$ et $WE_2$ est lié. Ce régime de couplage limite la résolution temporelle des signaux ampérométriques, ce qui perturbe les mesures de débit volumique $Q$ de la sueur circulant dans le canal microfluidique 11a, 11b ou 11c. Cet écueil est facilement évitable en ajustant les valeurs relatives de la distance inter-électrodes $L, L_a, L_b, L_c$ et de la durée $t_1 - t_0$ aux valeurs de la vitesse linéaire d'écoulement moyenne $V$ attendues.

Selon un premier mode de réalisation, illustré sur la [Fig.6], des couples de premières et de deuxièmes électrodes de travail $WE_1$ et $WE_2$ séparés par des distances inter-électrodes $L_a, L_b, L_c$ différentes peuvent être utilisés dans des canaux microfluidiques 11a, 11b, 11c parallèles séparés. De préférence, la distance inter-électrodes $L_a, L_b, L_c$ séparant les électrodes de travail $WE_1$ et $WE_2$ diffère en fonction du canal microfluidique 11a, 11b ou 11c considéré, par exemple telle que $L_a < L_b < L_c$.

En variante, selon un deuxième mode de réalisation illustré sur la [Fig.8], un réseau de deuxièmes électrodes de travail, ici une première deuxième électrode de travail $WE_2^{(1)}$ et une deuxième deuxième électrode de travail $WE_2^{(2)}$, peut être implémenté dans un même canal microfluidique 11, 11a, 11b, 11c. Selon des modes de réalisation non représentés, le réseau de deuxièmes électrodes de travail peut comprendre plus de deux deuxièmes électrodes de travail. Dans la suite, on se limitera à décrire le réseau de deuxièmes électrodes de travail implémenté dans le canal microfluidique référencé 11. Un tel réseau pourrait également être implémenté dans les canaux microfluidiques 11a, 11b, 11c.

Dans le canal microfluidique 11, chaque deuxième électrode de travail $WE_2^{(1)}$, $WE_2^{(2)}$ est respectivement disposée à une distance inter-électrodes $L^{(1)}$, $L^{(2)}$ différentes de la première électrode de travail $WE_1$. Les électrodes de travail $WE_1$, $WE_2^{(1)}$, $WE_2^{(2)}$ sont commutables électroniquement. La durée $t_1 - t_0$ est ajustée électroniquement par rétroaction de la valeur de vitesse linéaire d'écoulement moyenne $V$ mesurée lors des instants de mesure antérieurs.

Le débit volumique $Q$ peut ainsi être déterminé sur une large plage de valeurs, puisque la mesure de débit volumique $Q$ peut être effectuée dans chacun des canaux microfluidiques 11, 11a, 11b et 11c ou dans plusieurs d'entre eux, en ne retenant que les mesures de débit volumique $Q$ cohérentes avec les distances inter-électrodes $L_a, L_b, L_c, L^{(1)}, L^{(2)}$. Avantageusement, les distances inter-électrodes $L_a, L_b$ et $L_c, L^{(1)}, L^{(2)}$ sont de l'ordre du millimètre.

Les procédés de mesure du débit volumique $Q$ de sueur décrits ci-dessus peuvent être mis en œuvre de manière automatisée à l'aide d'un dispositif électronique de traitement 16, de préférence intégré à l'appareil 1.

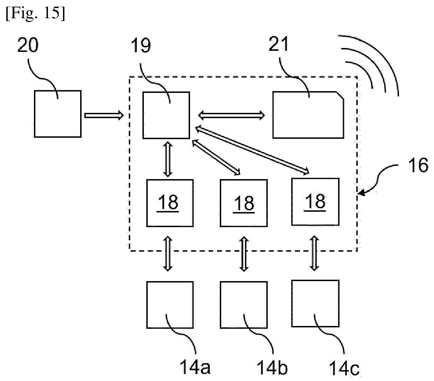

En référence à la [Fig.15], on décrit maintenant un mode de réalisation du dispositif électronique de traitement 16 pouvant être intégré dans l'appareil 1, par exemple sous la forme d'une carte électronique 17.

Dans le mode de réalisation à pluralité de canaux microfluidiques 11a, 11b, 11c représenté sur les figures 5 et 6, les cellules électrochimiques 14a, 14b, 14c sont connectées à un convertisseur analogique numérique 18, qui alimente lui-même un processeur 19. Le processeur 19 est par exemple programmé pour mettre en œuvre les procédés de mesure de débit volumique $Q$ de sueur décrits ci-dessus.

Dans le mode de réalisation représenté sur la [Fig.8] où sont implémentées dans un même canal microfluidique 11 une première électrode de travail $WE_1$ et plusieurs deuxièmes électrodes de travail $WE_2^{(1)}$, $WE_2^{(2)}$ situées respectivement à des distances inter-électrodes $L^{(1)}$ et $L^{(2)}$ différentes, chaque couple constitué par la première électrode de travail $WE_1$ et l'une des deuxièmes électrodes de travail $WE_2^{(1)}$, $WE_2^{(2)}$ forme une cellule électrochimique 14 connectée à un convertisseur analogique numérique 18, qui alimente lui-même un processeur 19. Le processeur 19 est par exemple programmé pour mettre en œuvre les procédés de mesure de débit volumique $Q$ de sueur décrits ci-dessus.

Une source d'énergie 20, par exemple une batterie, alimente le dispositif électronique de traitement 16. Un module de communication 21, filaire ou non filaire, peut aussi être prévu pour communiquer les résultats des mesures de débit volumique $Q$ de sueur à un appareil de stockage ou de post-traitement.

Le dispositif électronique de traitement 16 comporte éventuellement d'autres modules fonctionnels, par exemple un module gyroscopique et/ou accélérométrique pour détecter l'orientation et les mouvements du sujet 2, ainsi que pour quantifier son niveau d'activité, et/ou un capteur de température pour mesurer la température de l'épiderme du sujet 2. En effet, il est utile de connaître la température de la peau en raison des corrélations entre la température et le taux de sudation.

Certains éléments de l'appareil 1, notamment le dispositif électronique de traitement 16 peuvent être réalisés sous différentes formes, de manière unitaire ou distribuée, au moyen de composants matériels et/ou logiciels. Des composants matériels utilisables sont les circuits intégrés spécifiques (*Application-Specific In-tegrated Circuit* ou ASIC), les réseaux logiques programmables (*Field-Programmable Gate Array* ou FPGA). Des composants logiciels peuvent être écrits dans différents langages de programmation, par exemple C, C++, Java ou VHDL. Cette liste n'est pas exhaustive.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Dispositif électrochimique microfluidique (100) pour mesurer un débit volumique (Q) d'un fluide, le fluide comportant un solvant, le dispositif électrochimique microfluidique (100) comportant :

   - au moins un canal microfluidique (11, 11a, 11b, 11c) configuré pour permettre au fluide de s'écouler selon un sens d'écoulement (13) ;
   - au moins une cellule électrochimique (14, 14a, 14b, 14c) disposée dans l'au moins un canal microfluidique (11, 11a, 11b, 11c), la cellule électrochimique (14, 14a, 14b, 14c) comportant une première électrode de travail ($WE_1$) et au moins une deuxième électrode de travail ($WE_2$, $WE_2^{(1)}$, $WE_2^{(2)}$), ladite au moins une deuxième électrode de travail ($WE_2$, $WE_2^{(1)}$, $WE_2^{(2)}$) étant espacée de la première électrode de travail ($WE_1$) d'une distance inter-électrodes ($L_a$, $L_b$, $L_c$, $L^{(1)}$, $L^{(2)}$) dans le sens d'écoulement (13), au moins une contre-électrode (CE) et au moins une électrode de référence (REF) ; et
   - un système de mesure électrochimique par ampérométrie (15) configuré pour polariser la première électrode de travail ($WE_1$) à un premier potentiel d'électrode ($E_1$) et la deuxième électrode de travail ($WE_2$, $WE_2^{(1)}$, $WE_2^{(2)}$) à un deuxième potentiel d'électrode ($E_2$), de sorte que chacune desdites première et deuxième électrodes de travail produit un signal ampérométrique par réaction d'oxydation ou par réaction de réduction du solvant ou avec au moins une espèce chimique formant un couple d'oxydoréduction avec le solvant ;

   le système de mesure électrochimique par ampérométrie (15) étant configuré pour déterminer le débit volumique (Q) du fluide dans le canal microfluidique (11, 11a, 11b, 11c) à partir de la distance inter-électrodes ($L_a$, $L_b$, $L_c$, $L^{(1)}$, $L^{(2)}$) et d'un retard temporel ($\Delta t$) entre une variation du signal ampérométrique produit par la première électrode de travail ($WE_1$) et une variation du signal ampérométrique produit par la deuxième électrode de travail ($WE_2$, $WE_2^{(1)}$, $WE_2^{(2)}$).

2. Dispositif électrochimique microfluidique (100) selon la revendication 1, dans lequel le solvant est de l'eau $H_2O$.

3. Dispositif électrochimique microfluidique (100) selon la revendication 2, dans lequel le fluide est une sueur d'un sujet humain ou animal.

4. Dispositif électrochimique microfluidique (100) selon la revendication 2 ou 3, dans lequel le premier potentiel d'électrode ($E_1$) permet l'oxydation de l'eau $H_2O$ en dioxygène $O_2$ et le deuxième potentiel d'électrode $E_2$ permet la réduction du dioxygène $O_2$ dissous dans l'eau $H_2O$ produit en eau $H_2O$.

5. Dispositif électrochimique microfluidique (100) selon la revendication 2 ou 3, dans lequel le premier potentiel d'électrode ($E_1$) permet la réduction de l'eau $H_2O$ en dihydrogène $H_2$ et le deuxième potentiel d'électrode ($E_2$) permet la réduction de l'eau $H_2O$ en dihydrogène $H_2$.

6. Dispositif électrochimique microfluidique (100) selon la revendication 2 ou 3, dans lequel le premier potentiel d'électrode ($E_1$) permet la réduction du dioxygène $O_2$ dissous dans l'eau $H_2O$ en eau $H_2O$ et le deuxième potentiel d'électrode ($E_2$) permet la réduction du dioxygène $O_2$ dissous dans l'eau $H_2O$ en eau $H_2O$.

7. Dispositif électrochimique microfluidique (100) selon l'une des revendications 1 à 6, dans lequel le système de mesure électrochimique par ampérométrie (15) est également configuré pour :

   - au cours d'une première étape, polariser la première électrode de travail ($WE_1$) au premier potentiel d'électrode ($E_1$) et la deuxième électrode de travail ($WE_2$) au deuxième potentiel d'électrode ($E_2$) ;
   - au cours d'une deuxième étape, déconnecter la première électrode de travail ($WE_1$) ou fixer le premier potentiel d'électrode ($E_1$) à un potentiel proche ou égal d'un potentiel d'équilibre à courant nul.

8. Dispositif électrochimique microfluidique (100) selon l'une des revendications 1 à 7, comportant en outre un support isolant (4), ledit au moins un canal microfluidique (11, 11a, 11b, 11c) étant formé dans le support isolant (4), la première électrode de travail ($WE_1$) et ladite au moins une deuxième électrode de travail ($WE_2$, $WE_2^{(1)}$, $WE_2^{(2)}$) étant formées par des dépôts métalliques de platine ou de noir de platine sur ledit support isolant (6).

**9.** Dispositif électrochimique microfluidique (100) selon l'une des revendications 1 à 8, dans lequel la contre-électrode (CE) est positionnée en aval des électrodes de travail (WE$_1$, WE$_2$, WE$_2^{(1)}$, WE$_2^{(2)}$) selon le sens d'écoulement (13), et dans lequel l'électrode de référence (REF) est positionnée en amont desdites électrodes de travail (WE$_1$, WE$_2$, WE$_2^{(1)}$, WE$_2^{(2)}$) selon ledit sens d'écoulement (13).

**10.** Dispositif électrochimique microfluidique (100) selon l'une des revendications 1 à 9, comportant un premier et un deuxième canal microfluidique (11a, 11b), la première, respectivement, la deuxième, cellule électrochimique (14a, 14b) étant disposée dans le premier, respectivement, le deuxième, canal microfluidique (11a, 11b), la distance inter-électrodes ($L_a$) de la première cellule électrochimique (14a) étant différente de la distance inter-électrodes ($L_b$) de la deuxième cellule électrochimique (14b).

**11.** Dispositif électrochimique microfluidique (3) selon l'une des revendications 1 à 9, dans lequel ladite au moins une cellule électrochimique (14a) comporte deux deuxièmes électrodes de travail (WE$_2^{(1)}$, WE$_2^{(2)}$) respectivement séparées de la première électrode de travail (WE$_1$) par une première distance inter-électrode ($L^{(1)}$) et par une deuxième distance inter-électrode ($L^{(2)}$), la première distance inter-électrode ($L_a^{(1)}$) étant différente de la deuxième distance inter-électrode ($L^{(2)}$).

**12.** Dispositif électrochimique microfluidique (100) selon l'une des revendications 1 à 11, dans lequel le système de mesure électrochimique par ampérométrie (15) est configuré pour déterminer le débit volumique (Q) en fonction d'une surface de section (S) dudit canal microfluidique (11, 11a, 11b, 11c) selon le sens d'écoulement (13).

**13.** Appareil (1) destiné à être posé sur une zone d'investigation (8) d'un épiderme d'un sujet humain ou animal pour mesurer un paramètre quantitatif de sudation du sujet, ledit appareil (1) comportant :

- une structure définissant un dispositif électrochimique microfluidique (100) selon l'une des revendications 1 à 12, la structure comportant un orifice d'entrée (6) définissant la zone d'investigation (8) et laissant passer une sueur depuis l'épiderme, l'au moins un canal microfluidique (11, 11a, 11b, 11c) du dispositif électrochimique microfluidique (100) étant en communication avec l'orifice d'entrée (6) ; et
- un dispositif électronique de traitement (16) configuré pour déterminer le paramètre quantitatif de sudation dudit sujet humain ou animal à partir de mesures du débit volumique (Q) de sueur réalisées par le dispositif électrochimique microfluidique (100).

**14.** Appareil (1) selon la revendication 13, dans lequel le paramètre quantitatif de sudation dudit sujet humain ou animal est un taux de sudation.

**15.** Appareil (1) selon la revendication 13 ou 14, dans lequel la structure est une structure multicouche comportant une couche inférieure (3) et au moins une couche superposée sur la couche inférieure (3), le dispositif électrochimique microfluidique (100) s'étendant parallèlement à la couche inférieure (3), la couche inférieure (3) comprenant ledit orifice d'entrée (6).

**16.** Appareil (1) selon la revendication 15, dans lequel la structure multicouche comporte en outre une couche supérieure (10) et au moins une couche intermédiaire (4) située entre la couche inférieure (3) et la couche supérieure (10), le dispositif électrochimique microfluidique (100) étant formé dans l'épaisseur de l'au moins une couche intermédiaire (6).

**17.** Appareil (1) selon la revendication 16, dans lequel la couche supérieure (10) comporte un orifice de sortie (22) traversant la couche supérieure (10), et dans lequel l'au moins un canal microfluidique (11, 11a, 11b, 11c) est en communication avec l'orifice de sortie (22).

**18.** Appareil (1) selon l'une des revendications 16 ou 17, dans lequel la première électrode de travail (WE$_1$), l'au moins une deuxième électrode de travail (WE$_2$, WE$_2^{(1)}$, WE$_2^{(2)}$), ladite au moins une deuxième électrode de travail (WE$_2$, WE$_2^{(1)}$, WE$_2^{(2)}$), l'au moins une contre-électrode (CE) et l'au moins une électrode de référence (REF) sont disposées sur une face interne de la couche supérieure (10) fermant l'au moins un canal microfluidique (11, 11a, 11b, 11c) par le haut et/ou sur une face supérieure de la couche inférieure (3) fermant ledit au moins un canal microfluidique (11, 11a, 11b, 11c) par le bas.

**19.** Appareil (1) selon l'une des revendications 13 à 18, comportant en outre un dispositif de communication (21) configuré pour transmettre un ou plusieurs signaux de mesure produits par le dispositif électrochimique microfluidique (100).

**20.** Appareil (1) selon l'une des revendications 13 à 19, comportant en outre un module gyroscopique et/ou au moins un accéléromètre pour détecter un état d'activité dudit sujet humain ou animal.

**21.** Appareil (1) selon l'une des revendications 13 à 20, comportant en outre un capteur de température configuré pour mesurer la température de l'épiderme (2) dudit sujet humain ou animal.

**Patentansprüche**

**1.** Mikrofluidische elektrochemische Vorrichtung (100) zum Messen eines Volumenstroms (Q) einer Flüssigkeit, wobei die Flüssigkeit ein Lösungsmittel umfasst, wobei die mikrofluidische elektrochemische Vorrichtung (100) umfasst:

- mindestens einen mikrofluidischen Kanal (11, 11a, 11b, 11c), der so konfiguriert ist, dass die Flüssigkeit in einer Strömungsrichtung (13) fließen kann,
- mindestens eine elektrochemische Zelle (14, 14a, 14b, 14c), die in dem mindestens einen mikrofluidischen Kanal (11, 11a, 11b, 11c) angeordnet ist, wobei die elektrochemische Zelle (14, 14a, 14b, 14c) eine erste Arbeitselektrode ($WE_1$) und mindestens eine zweite Arbeitselektrode ($WE_2$, $WE_2^{(1)}$, $WE_2^{(2)}$), wobei die mindestens eine zweite Arbeitselektrode ($WE_2$, $WE_2^{(1)}$, $WE_2^{(2)}$) von der ersten Arbeitselektrode ($WE_1$) in Strömungsrichtung (13) um einen Elektrodenabstand ($L_a$, $L_b$, $L_c$, $L^{(1)}$, $L^{(2)}$) beabstandet ist, mindestens eine Gegenelektrode (CE) und mindestens eine Referenzelektrode (REF) umfasst, und
- ein elektrochemisches Messsystem mittels Amperometrie (15), das so konfiguriert ist, dass es die erste Arbeitselektrode ($WE_1$) auf ein erstes Elektrodenpotential ($E_1$) und die zweite Arbeitselektrode ($WE_2$, $WE_2^{(1)}$, $WE_2^{(2)}$) auf ein zweites Elektrodenpotential ($E_2$) polarisiert, so dass jede dieser ersten und zweiten Arbeitselektroden ein amperometrisches Signal durch Oxidationsreaktion oder durch Reduktionsreaktion des Lösungsmittels oder mit mindestens einer chemischen Spezies erzeugt, die mit dem Lösungsmittel ein Redox-Paar bildet,
- wobei das elektrochemische Messsystem mittels Amperometrie (15) so konfiguriert ist, dass es den Volumenstrom (Q) der Flüssigkeit in dem mikrofluidischen Kanal (11, 11a, 11b, 11c) aus dem Elektrodenabstand ($L_a$, $L_b$, $L_c$, $L^{(1)}$, $L^{(2)}$) und der Zeitverzögerung ($\Delta t$) zwischen der Änderung des von der ersten Arbeitselektrode ($WE_1$) erzeugten amperometrischen Signals und der Änderung des von der zweiten Arbeitselektrode ($WE_2$, $WE_2^{(1)}$, $WE_2^{(2)}$) erzeugten amperometrischen Signals.

**2.** Mikrofluidische elektrochemische Vorrichtung (100) nach Anspruch 1, wobei das Lösungsmittel Wasser $H_2O$ ist.

**3.** Mikrofluidische elektrochemische Vorrichtung (100) nach Anspruch 2, wobei die Flüssigkeit Schweiß eines menschlichen oder tierischen Subjekts ist.

**4.** Mikrofluidische elektrochemische Vorrichtung (100) gemäß Anspruch 2 oder 3, wobei das erste Elektrodenpotential ($E_1$) die Oxidation von Wasser $H_2O$ zu Sauerstoff $O_2$ ermöglicht und das zweite Elektrodenpotential $E_2$ die Reduktion des in Wasser $H_2O$ gelösten Sauerstoffs $O_2$ zu Wasser $H_2O$ ermöglicht.

**5.** Mikrofluidische elektrochemische Vorrichtung (100) gemäß Anspruch 2 oder 3, wobei das erste Elektrodenpotential ($E_1$) die Reduktion von Wasser $H_2O$ zu Wasserstoff $H_2$ ermöglicht und das zweite Elektrodenpotential ($E_2$) die Reduktion von Wasser $H_2O$ zu Wasserstoff $H_2$ ermöglicht.

**6.** Mikrofluidische elektrochemische Vorrichtung (100) gemäß Anspruch 2 oder 3, wobei das erste Elektrodenpotential ($E_1$) die Reduktion von in Wasser $H_2O$ gelöstem Sauerstoff $O_2$ zu Wasser $H_2O$ ermöglicht und das zweite Elektrodenpotential ($E_2$) die Reduktion von in Wasser $H_2O$ gelöstem Sauerstoff $O_2$ zu Wasser $H_2O$ ermöglicht.

**7.** Mikrofluidische elektrochemische Vorrichtung (100) gemäß einem der Ansprüche 1 bis 6, wobei das elektrochemische Messsystem mittels Amperometrie (15) auch so konfiguriert ist, dass es:

- in einem ersten Schritt die erste Arbeitselektrode ($WE_1$) auf das erste Elektrodenpotential ($E_1$) und die zweite Arbeitselektrode ($WE_2$) auf das zweite Elektrodenpotential ($E_2$) polarisiert,
- in einem zweiten Schritt die erste Arbeitselektrode ($WE_1$) trennt oder das erste Elektrodenpotential ($E_1$) auf ein Potential nahe oder gleich einem Nullstrom-Gleichgewichtspotential festlegt.

**8.** Mikrofluidische elektrochemische Vorrichtung (100) nach einem der Ansprüche 1 bis 7, die außerdem einen isolierenden Träger (4) umfasst, wobei der mindestens eine mikrofluidische Kanal (11, 11a, 11b, 11c) in dem isolierenden Träger (4) ausgebildet ist, wobei die erste Arbeitselektrode ($WE_1$) und die mindestens eine zweite Arbeitselektrode ($WE_2$, $WE_2^{(1)}$, $WE_2^{(2)}$) durch Metallablagerungen aus Platin oder Platinschwarz auf dem isolierenden Träger (6) gebildet sind.

9. Mikrofluidische elektrochemische Vorrichtung (100) nach einem der Ansprüche 1 bis 8, wobei die Gegenelektrode (CE) stromabwärts der Arbeitselektroden ($WE_1$, $WE_2$, $WE_2^{(1)}$, $WE_2^{(2)}$) in Strömungsrichtung (13) angeordnet ist und wobei die Referenzelektrode (REF) in Strömungsrichtung (13) stromaufwärts von den Arbeitselektroden ($WE_1$, $WE_2$, $WE_2^{(1)}$, $WE_2^{(2)}$) angeordnet ist.

10. Mikrofluidische elektrochemische Vorrichtung (100) nach einem der Ansprüche 1 bis 9, mit einem ersten und einem zweiten mikrofluidische Kanal (11a, 11b), wobei die erste bzw. zweite elektrochemische Zelle (14a, 14b) im ersten bzw. zweiten mikrofluidischen Kanal (11a, 11b) angeordnet ist, wobei der Elektrodenabstand ($L_a$) der ersten elektrochemischen Zelle (14a) sich vom Elektrodenabstand ($L_b$) der zweiten elektrochemischen Zelle (14b) unterscheidet.

11. Mikrofluidische elektrochemische Vorrichtung (3) nach einem der Ansprüche 1 bis 9, wobei die mindestens eine elektrochemische Zelle (14a) zwei zweite Arbeitselektroden ($WE_2^{(1)}$, $WE_2^{(2)}$) aufweist, die jeweils von der ersten Arbeitselektrode ($WE_1$) durch einen ersten Elektrodenabstand ($L^{(1)}$) und durch einen zweiten Elektrodenabstand ($L^{(2)}$) getrennt sind, wobei der erste Elektrodenabstand ($L^{(1)}$) sich vom zweiten Elektrodenabstand ($L^{(2)}$) unterscheidet.

12. Mikrofluidische elektrochemische Vorrichtung (100) nach einem der Ansprüche 1 bis 11, wobei das elektrochemische Messsystem mittels Amperometrie (15) so konfiguriert ist, dass es den Volumenstrom (Q) aus der Querschnittsfläche (S) des mikrofluidischen Kanals (11, 11a, 11b, 11c) in der Strömungsrichtung (13) bestimmt.

13. Gerät (1), bestimmt zum Aufsetzen auf einen Untersuchungsbereich (8) der Epidermis eines menschlichen oder tierischen Subjekts zum Messen eines quantitativen Parameters für das Schwitzen des Subjekts, wobei das Gerät(1) umfasst:

   - eine Struktur, die eine mikrofluidische elektrochemische Vorrichtung (100) gemäß einem der Ansprüche 1 bis 12 festlegt, wobei die Struktur eine Einlassöffnung (6) umfasst, die den Untersuchungsbereich (8) begrenzt und Schweiß aus der Epidermis durchlässt, wobei der mindestens eine mikrofluidische Kanal (11, 11a, 11b, 11c) der mikrofluidischen elektrochemischen Vorrichtung (100) mit der Einlassöffnung (6) in Verbindung steht, und
   - eine elektronische Verarbeitungsvorrichtung (16), die so konfiguriert ist, dass sie den quantitativen Parameter für das Schwitzen des menschlichen oder tierischen Subjekts aus Messungen des Schweißvolumenstroms (Q) bestimmt, die von der mikrofluidischen elektrochemischen Vorrichtung (100) durchgeführt werden.

14. Gerät (1) nach Anspruch 13, wobei der quantitative Parameter für das Schwitzen des Menschen oder Tieres eine Schwitzrate ist.

15. Gerät (1) nach Anspruch 13 oder 14, wobei die Struktur eine mehrschichtige Struktur ist, die eine untere Schicht (3) und mindestens eine auf der unteren Schicht (3) aufgebrachte Schicht umfasst, wobei sich die mikrofluidische elektrochemische Vorrichtung (100) parallel zur unteren Schicht (3) erstreckt, wobei die untere Schicht (3) die Einlassöffnung (6) umfasst.

16. Gerät (1) nach Anspruch 15, wobei die mehrschichtige Struktur ferner eine obere Schicht (10) und mindestens eine Zwischenschicht (4) zwischen der unteren Schicht (3) und der oberen Schicht (10) umfasst, wobei die mikrofluidische elektrochemische Vorrichtung (100) in der Dicke der mindestens einen Zwischenschicht (6) ausgebildet ist.

17. Gerät (1) nach Anspruch 16, wobei die obere Schicht (10) eine Auslassöffnung (22) aufweist, die die obere Schicht (10) durchdringt, und wobei der mindestens eine mikrofluidische Kanal (11, 11a, 11b, 11c) mit der Auslassöffnung (22) in Verbindung steht.

18. Gerät (1) nach einem der Ansprüche 16 oder 17, wobei die erste Arbeitselektrode ($WE_1$), die mindestens eine zweite Arbeitselektrode ($WE_2$, $WE_2^{(1)}$, $WE_2^{(2)}$), die mindestens eine zweite Arbeitselektrode ($WE_2$, $WE_2^{(1)}$, $WE_2^{(2)}$), die mindestens eine Gegenelektrode (CE) und die mindestens eine Referenzelektrode (REF) auf der Innenseite der oberen Schicht (10) angeordnet sind, die den mindestens einen mikrofluidischen Kanal (11, 11a, 11b, 11c) von oben verschließt, und/oder auf der Oberseite der unteren Schicht (3), die den mindestens einen mikrofluidischen Kanal (11,

**EP 4 548 049 B1**

11a, 11b, 11c) von unten verschließt.

19. Gerät (1) nach einem der Ansprüche 13 bis 18, das außerdem eine Kommunikationsvorrichtung (21) umfasst, die so konfiguriert ist, dass sie ein oder mehrere von der mikrofluidischen elektrochemischen Vorrichtung (100) erzeugte Messsignale überträgt.

20. Gerät (1) nach einem der Ansprüche 13 bis 19, das außerdem ein Gyroskopmodul und/oder mindestens einen Beschleunigungssensor umfasst, um den Aktivitätszustand des menschlichen oder tierischen Subjekts zu erfassen.

21. Gerät (1) nach einem der Ansprüche 13 bis 20, das außerdem einen Temperatursensor umfasst, der so konfiguriert ist, dass er die Temperatur der Epidermis (2) des menschlichen oder tierischen Subjekts misst.

**Claims**

1. A microfluidic electrochemical device (100) for measuring a volume flow rate (Q) of a fluid, the fluid comprising a solvent, the microfluidic electrochemical device (100) comprising:

   - at least one microfluidic channel (11, 11a, 11b, 11c) configured to allow the fluid to flow in a flow direction (13);
   - at least one electrochemical cell (14, 14a, 14b, 14c) disposed in the at least one microfluidic channel (11, 11a, 11b, 11c), the electrochemical cell (14, 14a, 14b, 14c) comprising a first working electrode ($WE_1$) and at least one second working electrode ($WE_2$, $WE_2^{(1)}$, $WE_2^{(2)}$), with said at least one second working electrode ($WE_2$, $WE_2^{(1)}$, $WE_2^{(2)}$) being spaced apart from the first working electrode ($WE_1$) by an inter-electrode distance ($L_a$, $L_b$, $L_c$, $L^{(1)}$, $L^{(2)}$) in the flow direction (13), at least one counter-electrode (CE) and at least one reference electrode (REF); and
   - an electrochemical amperometry measurement system (15) configured to bias the first working electrode ($WE_1$) at a first electrode potential ($E_1$) and the second working electrode ($WE_2$, $WE_2^{(1)}$, $WE_2^{(2)}$) at a second electrode potential ($E_2$), so that each of said first and second working electrodes produces an amperometric signal by oxidation reaction or by reduction reaction of the solvent or with at least one chemical species forming a redox couple with the solvent;

   the electrochemical amperometry measurement system (15) being configured to determine the volume flow rate (Q) of the fluid in the microfluidic channel (11, 11a, 11b, 11c) based on the inter-electrode distance ($L_a$, $L_b$, $L_c$, $L^{(1)}$, $L^{(2)}$) and a time delay ($\Delta t$) between a variation in the amperometric signal produced by the first working electrode ($WE_1$) and a variation in the amperometric signal produced by the second working electrode ($WE_2$, $WE_2^{(1)}$, $WE_2^{(2)}$).

2. The microfluidic electrochemical device (100) as claimed in claim 1, wherein the solvent is water $H_2O$.

3. The microfluidic electrochemical device (100) as claimed in claim 2, wherein the fluid is sweat from a human or animal subject.

4. The microfluidic electrochemical device (100) as claimed in claim 2 or 3, wherein the first electrode potential ($E_1$) allows the oxidation of water $H_2O$ to dioxygen $O_2$ and the second electrode potential $E_2$ allows the reduction of the dioxygen $O_2$ dissolved in the produced water $H_2O$ to water $H_2O$.

5. The microfluidic electrochemical device (100) as claimed in claim 2 or 3, wherein the first electrode potential ($E_1$) allows the reduction of water $H_2O$ to dihydrogen $H_2$ and the second electrode potential ($E_2$) allows the reduction of water $H_2O$ to dihydrogen $H_2$.

6. The microfluidic electrochemical device (100) as claimed in claim 2 or 3, wherein the first electrode potential ($E_1$) allows the reduction of dioxygen $O_2$ dissolved in water $H_2O$ to water $H_2O$ and the second electrode potential ($E_2$) allows the reduction of dioxygen $O_2$ dissolved in water $H_2O$ to water $H_2O$.

7. The microfluidic electrochemical device (100) as claimed in any of claims 1 to 6, wherein the electrochemical amperometry measurement system (15) is also configured to:

   - during a first step, bias the first working electrode ($WE_1$) at the first electrode potential ($E_1$) and the second working electrode ($WE_2$) at the second electrode potential ($E_2$);
   - during a second step, disconnect the first working electrode ($WE_1$) or set the first electrode potential ($E_1$) at a

potential close to or equal to a zero-current equilibrium potential.

8. The microfluidic electrochemical device (100) as claimed in any of claims 1 to 7, further comprising an isolating support (4), said at least one microfluidic channel (11, 11a, 11b, 11c) being formed in the isolating support (4), the first working electrode ($WE_1$) and said at least one second working electrode ($WE_2$, $WE_2^{(1)}$, $WE_2^{(2)}$) being formed by metal deposits of platinum or platinum black on said isolating support (6).

9. The microfluidic electrochemical device (100) as claimed in any of claims 1 to 8, wherein the counter-electrode (CE) is positioned downstream of the working electrodes ($WE_1$, $WE_2$, $WE_2^{(1)}$, $WE_2^{(2)}$) in the flow direction (13), and wherein the reference electrode (REF) is positioned upstream of said working electrodes ($WE_1$, $WE_2$, $WE_2^{(1)}$, $WE_2^{(2)}$) in said flow direction (13).

10. The microfluidic electrochemical device (100) as claimed in any of claims 1 to 9, comprising a first and a second microfluidic channel (11a, 11b), with the first, respectively, the second, electrochemical cell (14a, 14b) being disposed in the first, respectively, the second, microfluidic channel (11a, 11b), with the inter-electrode distance ($L_a$) of the first electrochemical cell (14a) being different from the inter-electrode distance ($L_b$) of the second electrochemical cell (14b).

11. The microfluidic electrochemical device (3) as claimed in any of claims 1 to 9, wherein said at least one electrochemical cell (14a) comprises two second working electrodes ($WE_2^{(1)}$, $WE_2^{(2)}$) respectively separated from the first working electrode ($WE_1$) by a first inter-electrode distance ($L^{(1)}$) and by a second inter-electrode distance ($L^{(2)}$), with the first inter-electrode distance ($L_a^{(1)}$) being different from the second inter-electrode distance ($L^{(2)}$).

12. The microfluidic electrochemical device (100) as claimed in any of claims 1 to 11, wherein the electrochemical amperometry measurement system (15) is configured to determine the volume flow rate (Q) as a function of a cross-sectional surface area (S) of said microfluidic channel (11, 11a, 11b, 11c) in the flow direction (13).

13. An apparatus (1) intended to be placed on an investigation zone (8) of an epidermis of a human or animal subject in order to measure a quantitative sweating parameter of the subject, said apparatus (1) comprising:

   - a structure defining a microfluidic electrochemical device (100) as claimed in any of claims 1 to 12, the structure comprising an inlet orifice (6) defining the investigation zone (8) and allowing through sweat from the epidermis, the at least one microfluidic channel (11, 11a, 11b, 11c) of the microfluidic electrochemical device (100) being connected to the inlet orifice (6); and
   - an electronic processing device (16) configured to determine the quantitative sweating parameter of said human or animal subject based on measurements of the volume flow rate (Q) of sweat carried out by the microfluidic electrochemical device (100).

14. The apparatus (1) as claimed in claim 13, wherein the quantitative sweating parameter of said human or animal subject is a sweating rate.

15. The apparatus (1) as claimed in claim 13 or 14, wherein the structure is a multi-layer structure comprising a lower layer (3) and at least one layer superimposed on the lower layer (3), with the microfluidic electrochemical device (100) extending parallel to the lower layer (3), the lower layer (3) comprising said inlet orifice (6).

16. The apparatus (1) as claimed in claim 15, wherein the multi-layer structure further comprises an upper layer (10) and at least one intermediate layer (4) located between the lower layer (3) and the upper layer (10), with the microfluidic electrochemical device (100) being formed within the thickness of the at least one intermediate layer (6).

17. The apparatus (1) as claimed in claim 16, wherein the upper layer (10) has an outlet orifice (22) passing through the upper layer (10), and wherein the at least one microfluidic channel (11, 11a, 11b, 11c) is connected to the outlet orifice (22).

18. The apparatus (1) as claimed in any of claims 16 or 17, wherein the first working electrode ($WE_1$), the at least one second working electrode ($WE_2$, $WE_2^{(1)}$, $WE_2^{(2)}$), the at least one counter-electrode (CE) and the at least one reference electrode (REF) are disposed on an inner face of the upper layer (10) closing the at least one microfluidic channel (11, 11a, 11b, 11c) from above and/or are disposed on an upper face of the lower layer (3) closing said at least one microfluidic channel (11, 11a, 11b, 11c) from below.

19. The apparatus (1) as claimed in any of claims 13 to 18, further comprising a communication device (21) configured to transmit one or more measurement signals produced by the microfluidic electrochemical device (100).

20. The apparatus (1) as claimed in any of claims 13 to 19, further comprising a gyroscopic module and/or at least one accelerometer for detecting a state of activity of said human or animal subject.

21. The apparatus (1) as claimed in any of claims 13 to 20, further comprising a temperature sensor configured to measure the temperature of the epidermis (2) of said human or animal subject.

[Fig. 1]

[Fig. 2]

[Fig. 3]

[Fig. 4]

[Fig. 5]

[Fig. 6]

[Fig. 7]

[Fig. 8]

[Fig. 9]

13

14, 14a, 14b, 14c

$H_2O$  $O_2$  $O_2$  $H_2O$

$WE_1$  $WE_2$

11, 11a, 11b, 11c

$L, L_a, L_b, L_c$

[Fig. 10]

101  $E_1$  $t$
0

102  $E_2$  $t$
0

103  $i_{ox}$  $t$
0

104  $i_{red}$  $t$
0
$\Delta t$
$t_0$  $t_1$

[Fig. 11]

[Fig. 12]

[Fig. 13]

13

14, 14a, 14b, 14c

$O_2$  $H_2O$          $O_2$  $H_2O$

$WE_1$                    $WE_2$

11, 11a, 11b,        $L, L_a, L_b, L_c$
11c

[Fig. 14]

$E_1$

0                                                                    $t$

141

$E_2$

$t$

142

$i_{red}$

0                                                                    $t$

143

$i_{red}$

0                                                                    $t$

144

$\Delta t$

$t_0$          $t_1$

[Fig. 15]

## RÉFÉRENCES CITÉES DANS LA DESCRIPTION

**Documents brevets cités dans la description**

- FR 3103901 A1
- WO 2021105122 A1